# EUROPEAN PATENT APPLICATION

(11) **EP 4 745 723 A1**
(43) Date of publication of application: **20.05.2026**
(21) Application number: 25212585.1
(22) Date of filing: 31.10.2025
(51) Int. Cl.: G06F 3/01, H04N 21/2343, H04N 21/6587

(54) **TECHNIQUES FOR REMOTELY MODIFYING VIDEO DATA QUALITY TO OPTIMIZE STREAMING IN SURGICAL EXTENDED REALITY**

(30) Priority: 15.11.2024 US 202463721329 P
(71) Applicant: Stryker Corporation, Portage, MI 49002 (US)
(72) Inventor: Scherf, Steven, Oakland, 94611 (US)
(74) Representative: Schott, Jakob Valentin

(57) **Abstract**

Extended reality surgical systems and methods involve a surgical device that includes a video source that is configured to generate video data including surgical content. A head-mounted device (HMD) includes an HMD display positionable in front of a user's eyes. The HMD is configured to receive the video data from a remote source and to stream the video data on the HMD display. The HMD acquires information indicative of the user's visual experience in consuming the video data. The information is transmitted to the remote source, and the remote source dynamically modifies at least one quality parameter of the video data based on the information and wirelessly transmits the modified video data to the HMD for streaming.

## Description

### CROSS-REFERENCE TO RELATED APPLICATIONS

The subject application claims priority to United States provisional patent application No. 63/721,329, filed November 15, 2024, the entire contents of which are hereby incorporated by reference.

### BACKGROUND

Extended reality is playing an increasingly important role in surgical guidance. For example, relevant surgical content can be displayed to an extended reality headset worn by the surgeon. The content can be superimposed onto the surgeon's direct view of the surgical site thereby enabling the surgeon to visualize the content without having to look away from the surgical site. Sometimes, the content is video content, e.g., obtained from a remote source or camera at the surgical site. Typically, the headset receives the content from the remote source via streaming over WiFi, for example.

Although extended reality has significant potential for improving surgery, there are limitations that need to be overcome. Surgical/medical use cases typically require streaming video to a headset to be performed in near-real time, while maintaining resolution and avoiding loss of frames. Meanwhile, wireless bandwidth is at a premium, as video streams, especially a 4K, 60FPS stream, are bandwidth hungry. The problem compounds when more than one stream is sent to the same headset or is streamed to multiple headsets in the room.

Normally, video sources are displayed on a standalone monitor in the operating room at full resolution because there is no bandwidth restriction when using a video cable. However, when streaming video content to an extended reality headset, conventional methods fail to optimize bandwidth and/or optimize video quality parameters to consume significantly less bandwidth. For instance, conventional methods fail to examine the user's virtual environment as the user consumes a video stream, and therefore, fail to detect situations in which the video quality can be eased back without noticeably impacting the experience of the user.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided, comprising: a surgical device that includes a video source that is configured to generate video data including surgical content; a head-mounted device (HMD) comprising an HMD display positionable in front of a user's eyes and wherein the HMD is configured to present, on the HMD display, a virtual window containing the video data; and one or more controllers being configured to: acquire spatial information related to one or both of: the virtual window relative to a field-of-view of the HMD display; and a gaze of the user relative to the virtual window; and dynamically modify at least one quality parameter of the video data based on the spatial information.

According to a second aspect, a method of operating a surgical system is provided, the surgical system includes a surgical device that has a video source, a head-mounted device (HMD) comprising an HMD display positionable in front of a user's eyes, and one or more controllers for performing the following steps: generating video data with the video source of the surgical device; presenting, on the HMD display, a virtual window containing the video data; acquiring spatial information related to one or both of: the virtual window relative to a field-of-view of the HMD display; and a gaze of the user relative to the virtual window; and dynamically modifying at least one quality parameter of the video data based on the spatial information.

Also provided are a non-transitory computer-readable medium comprising instructions, which when executed by one or more processors, operate the surgical system of the first aspect or second aspect.

According to a third aspect, a head-mounted device (HMD) is provided, comprising: an HMD display positionable in front of a user's eyes; an HMD controller coupled to the HMD display and being configured to: wirelessly receive video data including surgical content; present, on the HMD display, a virtual window containing the video data; acquire the spatial information related to one or both of: the virtual window relative to the field-of-view of the HMD display; and the gaze of the user relative to the virtual window; and dynamically modify the at least one quality parameter of the video data based on the spatial information.

Also provided are: a method of operating the HMD of the third aspect; and a non-transitory computer-readable medium comprising instructions, which when executed by one or more processors, operate the HMD of the third aspect.

According to a fourth aspect, a connectivity system for use with a surgical system that includes video data including surgical content, and a head-mounted device that includes an HMD controller and an HMD display positionable in front of a user's eyes, the HMD display being configured to present a virtual window containing the video data, the connectivity system comprising: a controller configured to: receive the video data from the surgical system; wirelessly receive spatial information from the HMD controller, the spatial information related to one or both of: a virtual window relative to the field-of-view of the HMD display; and the gaze of the user relative to the virtual window; dynamically modify the at least one quality parameter of the video data based on the spatial information; and wirelessly transmit the modified video data to the HMD controller.

Also provided are: a method of operating the connectivity system of the fourth aspect; a non-transitory computer-readable medium comprising instructions, which when executed by one or more processors, operate the connectivity system of the fourth aspect; a host system/device including the connectivity system of the fourth aspect; a method of operating the host system/device including the connectivity system of the fourth aspect; an extended reality system including the connectivity system of the fourth aspect; a method of operating the extended reality system including the connectivity system of the fourth aspect; an HMD including the connectivity system of the fourth aspect; a method of operating the HMD including the connectivity system of the fourth aspect.

According to a fifth aspect, a surgical system is provided, comprising: a head-mounted device (HMD) comprising an HMD display positionable in front of a user's eyes; a remote source that is configured to generate video data including surgical content and wirelessly transmit the video data to the HMD; wherein the HMD is configured to: wirelessly receive the video data from the remote source and stream the video data on the HMD display; and acquire information indicative of the user's visual experience relative to the video data; and wirelessly transmit the information to the remote source; and wherein the remote source is configured to: wirelessly receive the information from the HMD; dynamically modify at least one quality parameter of the video data based on the information; and wirelessly transmit the modified video data to the HMD for streaming on the HMD display.

According to a sixth aspect, a surgical system is provided comprising: a surgical device that includes a video source that is configured to generate video data including surgical content; a head-mounted device (HMD) comprising an HMD display positionable in front of a user's eyes and wherein the HMD is configured to present, on the HMD display, a virtual window containing the video data; and one or more controllers being configured to: automatically detect contextual information related to the surgical content of video data presented in the virtual window; and dynamically modify at least one quality parameter of the video data based on the contextual information.

According to a seventh aspect a surgical system is provided, comprising: a surgical device that includes a video source that is configured to generate video data including surgical content; a head-mounted device (HMD) comprising an HMD display positionable in front of a user's eyes and wherein the HMD is configured to present, on the HMD display, a virtual window containing the video data; and one or more controllers being configured to: detect quantitative and/or qualitative information related to the video data presented in the virtual window; and dynamically modify at least one quality parameter of the video data based on the quantitative and/or qualitative information.

Also provided are: an HMD of the fifth, sixth, or seventh aspect; a connectivity system of the fifth, sixth, or seventh aspect; a method of operating the surgical system of the fifth, sixth, or seventh aspect; a method of operating the HMD of the fifth, sixth, or seventh aspect; a method of operating the connectivity system of the fifth, sixth, or seventh aspect; and a non-transitory computer-readable medium (or computer program product) comprising instructions, which when executed by one or more processors, operate the capabilities of the fifth, sixth, or seventh aspect.

According to an eighth aspect, a connectivity system is provided for use with a surgical system that includes video data including surgical content, and a head-mounted device that includes an HMD controller and an HMD display positionable in front of a user's eyes, the connectivity system comprising: a controller configured to: receive the video data from the surgical system; detect information related to the contents, context, and/or quality of the video data; dynamically modify at least one quality parameter of the video data based on the detected information; and wirelessly transmit the modified video data to the HMD for streaming on the HMD display.

Any of the above aspects may be combined, in whole or in part.

Any of the above aspects may be combined with any of the following implementations. Any of the following implementations may be utilized in part, or in whole, with any of the above aspects. The implementations include, but are not limited to:

The quality parameter of the video data can be a parameter of: resolution, compression, bitrate, target bitrate, constant bitrate, variable bitrate, frame rate, resolution, group of picture (GOP) key frame size, profile and level, B-frame, reference frames, entropy coding, chroma subsampling, intra refresh, deblocking filter, tuning, encoding speed or the like. The quality parameter of the video data can be dynamically decreased or increased, given the specific conditions.

The information can be information related to the user's experience in viewing or consuming the video data VD. In some cases, the information actually detects data derived from the user's visual experience. In other cases, the information may be used to infer or predict what the user may be visually experiencing. In other cases, the information may not be related to the user's visual experience. The information can be spatial information related to the virtual window relative to the field-of-view of the HMD display. The controller(s) can: determine a size of the virtual window relative to the field-of-view; dynamically modify the at least one quality parameter of the video data based on the size of the virtual window; dynamically modify the at least one quality parameter to increase quality of the video data in response to detecting an increase in the size of the virtual window; dynamically modify the at least one quality parameter to decrease quality of the video data in response to detecting a decrease in the size of the virtual window; and/or dynamically modify the resolution of the video data proportional to the size of the virtual window. The information indicative of the user's visual experience relative to the video data comprises information related to one or more of the following: a size of the video data relative to a field-of-view of the HMD display; a location of the video data relative to a field-of-view of the HMD display; a gaze of the user relative to the video data; a gaze of the user identifying that the user is focused on the video data; a gaze of the user identifying that the user is focused on a second video data presented on the HMD display; a gaze of the user identifying that the user is focused on a sub-region of interest of the video data; a gaze of the user identifying that the user is focused on a specific object of interest of the video data; a detected object in the video data presented to the user; contents of the video data presented to the user; context of the video data presented to the user; a qualitative measure of the video data presented to the user; and a quantitative measure of the video data presented to the user.

The information can be spatial information related to the gaze of the user relative to the virtual window. The controller(s) can acquire the spatial information related to the gaze of the user to identify a sub-region of interest of the virtual window that the user is focused on and identify a remaining region of the virtual window that the user is not focused on. The controller(s) can dynamically modify the at least one quality parameter to increase quality of the video data in the region of interest of the virtual window; and/or dynamically modify the at least one quality parameter to decrease quality of the video data in the remaining region of the virtual window. The controller(s) can acquire spatial information related to the gaze of the user to identify an object of interest of the virtual window that the user is focused on and identify a remaining region of the virtual window that the user is not focused on. The controller(s) can dynamically modify the at least one quality parameter of the video data in a region defining the object of interest of the virtual window. The controller(s) can acquire the spatial information to identify that the gaze of the user is focused on the virtual window containing the video data and to identify that the user is not focused on the other content. The controller(s) can, in response, dynamically modify the at least one quality parameter to increase quality of the video data in the virtual window. The controller(s) can acquire the spatial information to identify that the gaze of the user is focused on the other content and to identify that the user is not focused on the virtual window containing the video data. The controller(s) can, in response, dynamically modify the at least one quality parameter to decrease quality of the video data in the virtual window.

The information can be contextual information acquired based on the surgical contents of the video data after presentation in the virtual window or before presentation in the virtual window. Detection of contextual information can be based on one or more of: a surgical step; an aspect of a surgical step; a portion of a graphical user interface; a surgical task requiring attention by the HMD user; a critical anatomical structure; presence of a surgical tool or object; an alert or warning, or the like. The information can be qualitive information related to the video data. For example, the qualitive information can be related to any of the described quality parameters. The information can be quantitative information related to the video data. The one or more controllers can employ a machine learning model to predict the most appropriate information to acquire. The one or more controllers can employ a machine learning model to predict the most manner to modify the quality parameter of the video data.

The surgical device can include: a surgical scope comprising a camera as the video source; a surgical robot or surgical tool comprising a camera as the video source; a navigation system comprising a camera as the video source; a second HMD comprising a camera as the video source; an ultrasound scanner coupled to the video source; a computing device that runs a clinical application. The surgical content can include a real-world view of a surgical site and/or a virtual representation of a surgical site. The surgical content can be live or recorded. The HMD can be configured to present, on the HMD display, the virtual window containing a live version of the video data or a replay of the video data. The remote source can be any of the described surgical devices or a connectivity system that is coupled to the surgical device and configured to wirelessly communicate with the HMD.

### BRIEF DESCRIPTION OF THE DRAWINGS

Advantages of the present invention will be readily appreciated as the same becomes better understood by reference to the following detailed description when considered in connection with the accompanying drawings wherein:
FIG. 1 is a perspective view of a surgical system, according to one implementation.
FIG. 2 is a schematic view of an example control system that can be used with the surgical system.
FIG. 3 is an illustration of various coordinate systems and transforms that can be established relative to the various components of the surgical system, according to one implementation.
FIGS. 4 is a sequence diagram illustrating an example method related to remotely modifying quality parameter(s) of video data that is transmitted to an HMD for streaming, according to one implementation.
FIGS. 5A and 5B illustrate a first-person view of the HMD user whereby the quality of the video data is modified based on the relative size of the video data increasing due to HMD user moving the video data closer using gestures, according to one example.
FIGS. 6A and 6B illustrate a first-person view of the HMD user whereby the quality of the video data is modified based on the relative size of anchored video data increasing due to HMD user moving closer the anchored video data, according to one example.
FIGS. 7A and 7B illustrate a first-person view of the HMD user whereby the quality of the video data is modified depending on whether the HMD user is gazing at the video data or surrounding environment, according to one example.
FIGS. 8A and 8B illustrate a first-person view of the HMD user whereby the quality of a first video data window or second video data window is modified depending on whether the HMD user is gazing at the first video data window or the second video data window, according to one example.
FIGS. 9A and 9B illustrate a first-person view of the HMD user whereby the quality of a sub-region of the video data or surrounding environment outside of the sub-region is modified depending on whether the HMD user is gazing at the sub-region or surrounding environment, according to one example.

### DETAILED DESCRIPTION

### I. Example System Overview

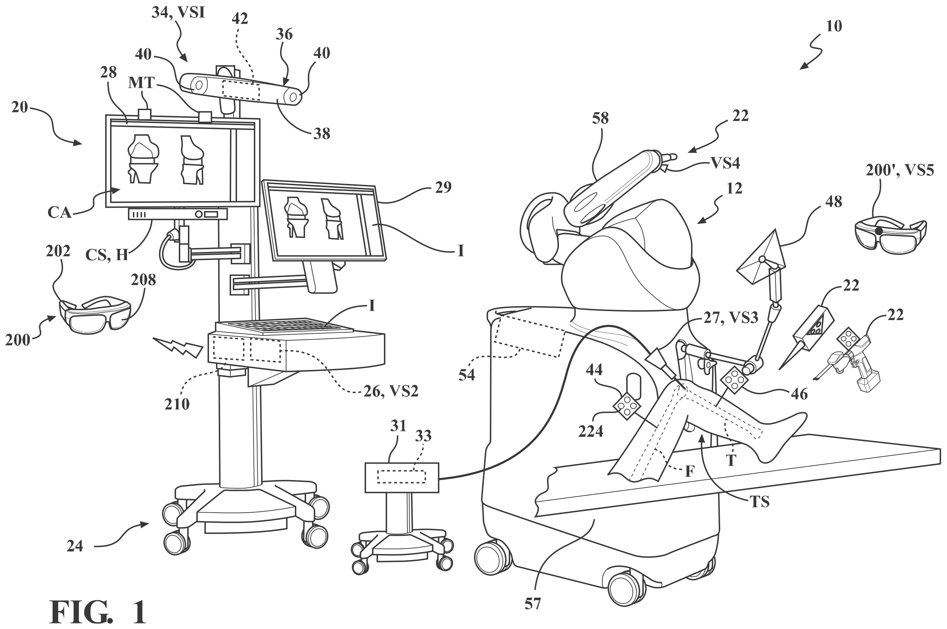

Referring to FIG. 1, a system 10 is provided. The system can be a surgical system 10 adapted for treating a patient. The surgical system 10 is shown in a surgical setting such as an operating room of a medical facility. The surgical system 10 may be used to perform any intraoperative surgical procedure on a patient. Example surgical procedures include, but are not limited to: partial knee arthroplasty, total knee arthroplasty, total hip arthroplasty, shoulder arthroplasty, spinal procedures, ankle procedures, endoscopic procedures, cranial procedures, lesion removal procedures, arthroscopic procedures, arthroscopic resection procedures, soft tissue or ligament repair procedures, neurological procedures, ENT procedures, minimally invasive MIS procedures, or the like. In the example shown in FIG. 1, the patient is undergoing a knee procedure. For example, the surgical system 10 can be used for performing an arthroplasty procedure in which material is removed from a femur F and/or a tibia T of a patient. However, it should be recognized that the surgical system 10 may be used to perform any suitable procedure in which material is removed from any suitable portion of a patient's anatomy, material is added to any suitable portion of the patient's anatomy (e.g., an implant, graft, etc.), and/or in which any other control of and/or visualization of a surgical tool is desired.

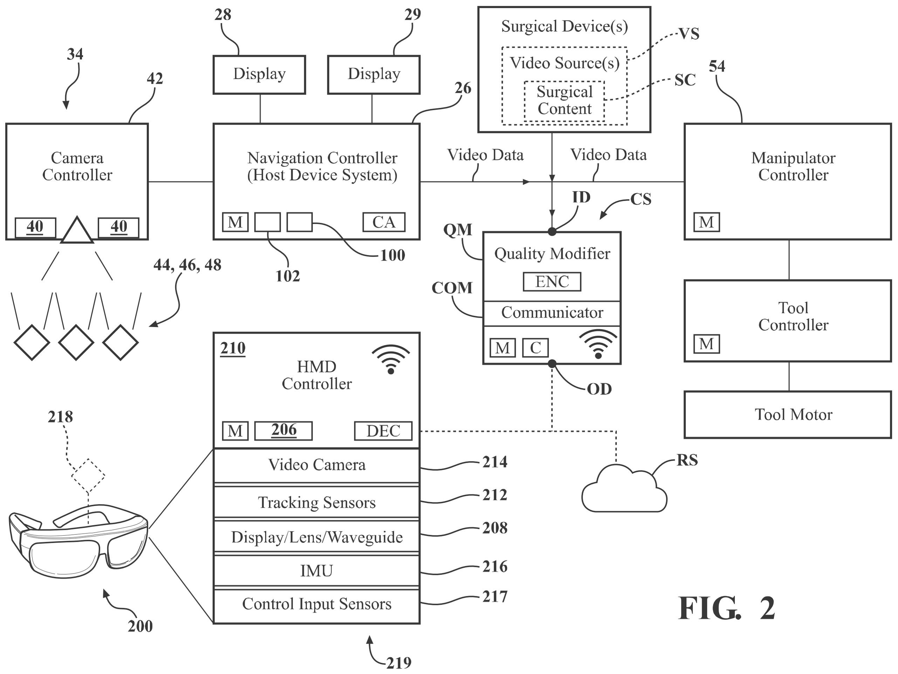

In the implementation shown, the surgical system 10 includes a manipulator 12 (e.g., surgical robot) and a navigation system 20. The navigation system 20 is configured to track movement of various objects in the operating room. Such objects include, for example, a surgical tool 22, a target site (TS) of the anatomy of the patient (e.g., femur F and tibia T). The navigation system 20 tracks these objects and can display their relative positions and orientations to the surgeon on a clinical application (CA) and, in some cases, for purposes of controlling or constraining movement of the surgical tool 22 relative to virtual cutting boundaries (VB) associated with the target site (TS). An example control scheme for the surgical system 10 is shown in FIG. 2.

In the implementation shown, the surgical tool 22 is attached to the manipulator 12. Such an arrangement is shown in U.S. Patent No. 9,119,655, entitled, "Surgical Manipulator Capable of Controlling a Surgical Instrument in Multiple Modes," the disclosure of which is hereby incorporated by reference. In one example, the manipulator 12 has a base 57, a plurality of links 58 extending from the base 57, and a plurality of joints for moving the surgical tool 22 with respect to the base 57. The links 58 and joints form a robotic arm. Some or all of the joints may be passive joints or active joints. The manipulator 12 may have a serial arm or parallel arm configuration. The manipulator 12 can be floor mounted, ceiling mounted, gantry mounted, table mounted, or patient mounted. More than one manipulator 12 can be utilized.

While the surgical system 10 is illustrated in FIGS. 1-3 as including the surgical tool 22 attached to the manipulator 12, it should be recognized that the surgical system 10 may additionally or alternatively include one or more manually operated or hand-held surgical tools 22. For example, the surgical tool 22 may include a hand-held motorized saw, drill, bur, probe, or other suitable tool that may be held and manually operated by a surgeon. Any implementations described with reference to the use of the manipulator 12 may also apply to the use of a hand-held tool 22 with appropriate modifications.

The navigation system 20 includes one or more computer cart assemblies 24 that houses one or more navigation controllers 26. A navigation interface is in operative communication with the navigation controller 26. The navigation interface includes one or more displays 28, 29 adjustably mounted to the computer cart assembly 24 or mounted to separate carts as shown. Input devices, such as a keyboard and mouse can be used to input information into the navigation controller 26 or otherwise select/control certain aspects of the navigation controller 26. Other input devices are contemplated including a touch screen, a microphone for voice-activation input, an optical sensor for gesture input, and the like.

The clinical application CA can be displayed on one or more displays 28, 29 of the navigation system 20. The clinical application CA assists a surgeon or staff in performing the surgical procedure. The clinical application CA can have a plurality of different screens related to the surgical procedure. Such screens can include a pre-operative planning screen, an operating room setup screen, an anatomical registration screen, an intra-operative planning screen, an anatomical preparation screen, or a post-operative evaluation screen, and the like. The clinical application CA can present a medical imaging data that is preoperative acquired or intraoperatively acquired. The clinical application CA can also present a navigation guidance region that displays one or more of the surgical objects tracked by a localizer 34 of the navigation system 20.

The localizer 34 communicates with the navigation controller 26. In one implementation, as shown, the localizer 34 is an optical localizer and includes a camera unit 36. The camera unit 36 has a housing 38 comprising an outer casing that houses one or more optical sensors 40. The optical sensors 40 can detect light signals, such as infrared (IR) signals and/or visible light signals. Camera unit 36 can be mounted on an adjustable arm to position the optical sensors 40 with a field-of-view of the below discussed trackers that, ideally, is free from obstructions. The camera unit 36 includes a camera controller 42 in communication with the optical sensors 40 to receive signals from the optical sensors 40. The camera controller 42 communicates with the navigation controller 26 through either a wired or wireless connection (not shown). In other implementations, the optical sensors 40 communicate directly with the navigation controller 26. Position and orientation signals and/or data are transmitted to the navigation controller 26 for purposes of tracking objects. The computer cart assembly 24, display 28, and camera unit 36 may be like those described in U.S. Patent No. 7,725,162 to Malackowski, et al. issued on May 25, 2010, entitled "Surgery System," the disclosure of which is hereby incorporated by reference. The navigation controller 26 can be a personal computer or laptop computer. Navigation controller 26 includes a central processing unit (CPU) and/or other processors, memory (not shown), and storage (not shown). The navigation controller 26 is loaded with software that converts the signals received from the camera unit 36 into data representative of the position and orientation of the objects being tracked. The navigation controller 26 includes a navigation processor. It should be understood that the navigation processor could include one or more processors to control operation of the navigation controller 26. The processors can be any type of microprocessor or multi-processor system. The term processor is not intended to limit the scope of any implementation to a single processor.

Navigation system 20 is operable with a plurality of tracking devices 44, 46, 48, also referred to herein as trackers. In the illustrated implementation, one tracker 44 can be an anatomical tracker, e.g., firmly affixed to the femur F of the patient and another tracker 46 can be firmly affixed to the tibia T of the patient. Trackers 44, 46 are firmly affixed to sections of bone in an implementation. For example, trackers 44, 46 may be attached to the bone in the manner shown in U.S. Patent No. 7,725,162 to Malackowski, et al. issued on May 25, 2010, entitled "Surgery System," the disclosure of which is hereby incorporated by reference. Trackers 44, 46 may also be mounted like those shown in U.S. Patent Application No. 14/156,856, filed on January 16, 2014, entitled, "Navigation Systems and Methods for Indicating and Reducing Line-of-Sight Errors," hereby incorporated by reference herein. The trackers 44, 46 may be mounted to other tissue types or parts of the anatomy. A tool tracker 48 can be coupled to the manipulator 12 or the tool 22 at any suitable location. The tool tracker 48 can be integrated into the surgical tool 22 during manufacture or may be separately mounted to the surgical tool 22 (or to an end effector attached to the manipulator 12 of which the surgical tool 22 forms a part) in preparation for surgical procedures. The working end of the surgical tool 22, which is being tracked by virtue of the tool tracker 48, may be referred to herein as an energy applicator, and may be a rotating bur, saw, router, reamer, impactor, electrical ablation device, cut guide, tool holder, probe, or the like.

In one implementation, optical sensors 40 of the localizer 34 receive light signals from the trackers 44, 46, 48. In one example, the trackers 44, 46, 48 are passive trackers. In this implementation, each tracker 44, 46, 48 has at least three passive tracking elements or markers (e.g., reflectors) for transmitting light signals (e.g., reflecting light emitted from the camera unit 36) to the optical sensors 40. In other implementations, active tracking markers can be employed. The active markers can be, for example, light emitting diodes transmitting light, such as infrared light. Active and passive arrangements are possible. The camera unit 36 receives optical signals from the trackers 44, 46, 48 and outputs to the navigation controller 26 signals relating to the position of the tracking markers of the trackers 44, 46, 48 relative to the localizer 34. Based on the received optical signals, navigation controller 26 generates data indicating the relative positions and orientations of the trackers 44, 46, 48 relative to the localizer 34. These relative positions can be displayed on the clinical application CA as graphical representations for surgical guidance.

In another implementation, the navigation system 20 and/or the localizer 34 are radio frequency (RF) based. For example, the navigation system 20 may comprise an RF transceiver coupled to the navigation controller 26. Here, the trackers 44, 46, 48 may comprise RF emitters or transponders, which may be passive or may be actively energized. The RF transceiver transmits an RF tracking signal, and the RF emitters respond with RF signals such that tracked states are communicated to (or interpreted by) the navigation controller 26. The RF signals may be of any suitable frequency. The RF transceiver may be positioned at any suitable location to track the objects using RF signals effectively. Furthermore, examples of RF-based navigation systems may have structural configurations that are different than the navigation system 20 illustrated throughout the drawings.

In other examples, the navigation system 20 and/or localizer 34 are electromagnetically (EM) based. For example, the navigation system 20 may comprise an EM transceiver coupled to the navigation controller 26. Here, the trackers 44, 46, 48 may comprise EM components attached thereto (e.g., various types of magnetic trackers, electromagnetic trackers, inductive trackers, and the like), which may be passive or may be actively energized. The EM transceiver generates an EM field, and the EM components respond with EM signals such that tracked states are communicated to (or interpreted by) the navigation controller 26. The navigation controller 26 may analyze the received EM signals to associate relative states thereto. Here too, examples of EM-based navigation systems may have structural configurations that are different than the navigation system 20 illustrated throughout the drawings.

In other examples, the navigation system 20 and/or the localizer 34 could be based on one or more other types of tracking systems. For example, an ultrasound-based tracking system coupled to the navigation controller 26 could be provided to facilitate acquiring ultrasound images of markers that define trackable features on the tracked objects such that tracked states are communicated to (or interpreted by) the navigation controller 26 based on the ultrasound images. By way of further example, a fluoroscopy-based imaging system (e.g., a C-arm) coupled to the navigation controller 26 could be provided to facilitate acquiring X-ray images of radio-opaque markers that define trackable features such that tracked states are communicated to (or interpreted by) the navigation controller 26 based on the X-ray images.

Furthermore, in some examples, a machine-vision tracking system, including a vision camera can be coupled to the navigation controller 26 and could be provided to facilitate acquiring 2D and/or 3D machine-vision images of structural features that define trackable features such that tracked states TS are communicated to (or interpreted by) the navigation controller 26 based on the machine-vision images. The machine vision system can be integrated into the camera unit 36, optionally in combination with infrared sensors. The machine vision system can create depth maps and can detect objects with or without trackers. The machine vision system can detect patterns, shapes, colors, computer-codes, tracking geometries, and the like.

Various types of tracking and/or imaging systems could define the localizer 34 and/or form a part of the navigation system 20 without departing from the scope of the present disclosure. Furthermore, the navigation system 20 and/or localizer 34 may have other suitable components or structure not specifically recited herein, and the various techniques, methods, and/or components described herein with respect to the optically-based navigation system 20 shown throughout the drawings may be implemented or provided for any of the other examples of the navigation system 20 described herein. For example, the navigation system 20 may utilize solely inertial tracking and/or combinations of different tracking techniques, sensors, and the like. Other configurations are contemplated.

Based on the position and orientation of the trackers 44, 46, 48 and previously loaded data, navigation controller 26 can determine the position of the working end of the surgical tool 22 (e.g., the centroid of a surgical bur) and/or the orientation of the surgical tool 22 relative to the tissue against which the working end is to be applied. In some implementations, the navigation controller 26 forwards these data to a manipulator controller 54. The manipulator controller 54 can then use the data to control the manipulator 12. This control can be like that described in U.S. Patent No. 9,119,655, entitled, "Surgical Manipulator Capable of Controlling a Surgical Instrument in Multiple Modes," or like that described in U.S. Patent No. 8,010,180, entitled, "Haptic Guidance System and Method", the disclosures of which are hereby incorporated by reference.

In one implementation, the manipulator 12 is controlled to stay within a preoperatively defined virtual boundary VB that can be determined by a surgical plan. The virtual boundary VB may be a virtual cutting boundary which defines the material of the anatomy (e.g., the femur F and tibia T) to be removed by the surgical tool 22. More specifically, each of the femur F and tibia T has a target volume of material that is to be removed by the working end of the surgical tool 22. The target volumes are defined by one or more virtual cutting boundaries. The virtual cutting boundaries define the surfaces of the bone that should remain after the procedure. The navigation system 20 tracks and controls the surgical tool 22 to ensure that the working end, e.g., the surgical bur, removes the target volume of material and does not extend beyond the virtual cutting boundary, as disclosed in U.S. Patent No. 9,119,655, entitled, "Surgical Manipulator Capable of Controlling a Surgical Instrument in Multiple Modes," the disclosure of which is hereby incorporated by reference, or as disclosed in U.S. Patent No. 8,010,180, entitled, "Haptic Guidance System and Method", the disclosure of which is hereby incorporated by reference.

The virtual cutting boundary VB may be defined within a virtual model of the anatomy (e.g., the femur F and tibia T), or separately from the virtual model. The virtual cutting boundary may be represented as a mesh surface, constructive solid geometry (CSG), voxels, or using other boundary representation techniques. The surgical tool 22 may be used to cut away material from the femur F and tibia T to receive an implant. The surgical implants may include unicompartmental, bicompartmental, or total knee implants as shown in U.S. Patent No. 9,381,085, entitled, "Prosthetic Implant and Method of Implantation," the disclosure of which is hereby incorporated by reference. Other implants, such as hip implants, shoulder implants, spine implants, and the like are also contemplated. The focus of the description on knee implants is provided as one example. These concepts can be equally applied to other types of surgical procedures, including those performed without placing implants.

The navigation controller 26 also generates image signals that indicate the relative position of the working end to the tissue. These image signals are applied to the displays 28, 29. The displays 28, 29, based on these signals, generate images on the clinical application CA that allow the surgeon and staff to view the relative position of the working end to the target site TS.

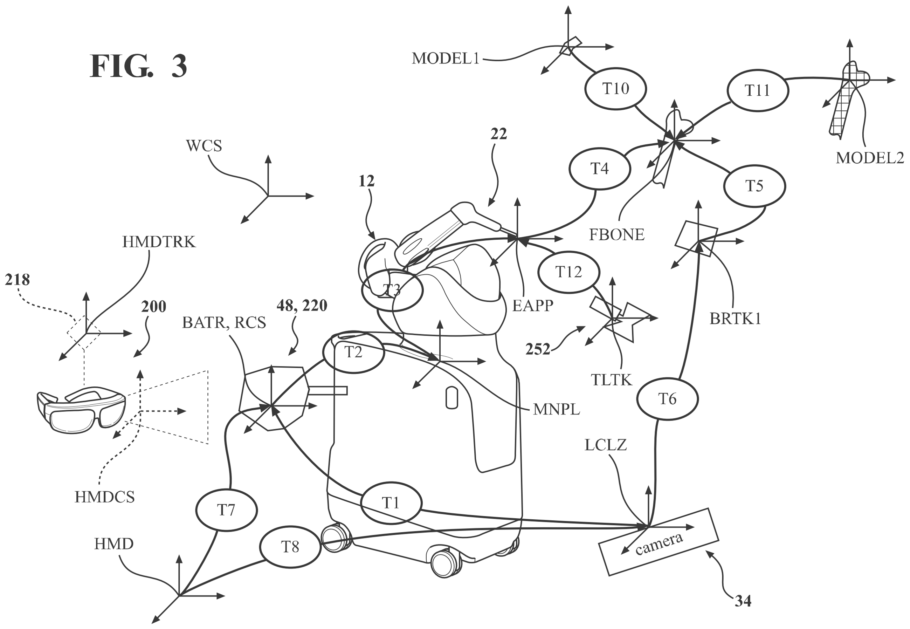

Referring to FIG. 3, tracking of objects can be conducted with reference to a localizer coordinate system LCLZ. The localizer coordinate system has an origin and an orientation (a set of x, y, and z planes). Each tracker 44, 46, 48 and object being tracked also has its own coordinate system separate from the localizer coordinate system LCLZ. Components of the navigation system 20 that have their own coordinate systems are the bone trackers 44, 46 (one of which is shown in FIG. 3) and the base tracker 48. These coordinate systems are represented as, respectively, bone tracker coordinate systems BTRK1, BTRK2 (BTRK1 shown), and base tracker coordinate system BATR. The world coordinate system WCS indicates the coordinate system of the real-world, or room, in which the objects are located.

Navigation system 20 monitors the positions of the femur F and tibia T of the patient by monitoring the position of bone trackers 44, 46 rigidly attached to bone. Femur coordinate system is FBONE and tibia coordinate system is TBONE, which are the coordinate systems of the bones to which the bone trackers 44, 46 are rigidly attached.

Prior to the start of the intraoperative procedure, preoperative images of the target site (TS) may be generated (or of other portions of the anatomy in other implementations). The preoperative images can be stored as two-dimensional or three-dimensional patient image data in a computer-readable storage device, such as memory within the navigation system 20. The patient image data may be based on X-ray scans or computed tomography (CT) scans of the patient's anatomy. The patient image data may then be used to generate two-dimensional images or three-dimensional models of the patient's anatomy. The pre-operative data and models may be used for purposes of surgical planning purposes and intraoperative guidance. For example, the surgical plan (e.g., tool path TP or resection volume or boundaries VB), may be planned relative to the virtual model. The virtual model and surgical plan can then be registered to the anatomy using any appropriate registration technique, such as pointer registration, imageless registration, or the like.

In preparation for the intraoperative procedure, the images or three-dimensional models developed from the image data are mapped to the anatomy coordinate system, e.g., femur coordinate system FBONE and tibia coordinate system TBONE (see transform T11). One of these models is shown in FIG. 3 with model coordinate system MODEL2. These images/models are fixed in the femur coordinate system FBONE and tibia coordinate system TBONE. As an alternative to taking preoperative images, modeling and plans for treatment can be developed intraoperatively and "on the fly" in operating room (OR) from using the navigation pointer 22, bone tracing, and other methods. The models described herein may be represented by mesh surfaces, constructive solid geometry (CSG), voxels, or other model constructs.

During an initial phase of the intraoperative procedure, the bone trackers 44, 46 are coupled to the bones of the patient. The pose (position and orientation) of coordinate systems FBONE and TBONE are mapped to coordinate systems BTRK1 and BTRK2, respectively (see transform T5). In one implementation, a pointer instrument 252 (TLTK), such as disclosed in U.S. Patent No. 7,725,162 to Malackowski, et al., hereby incorporated by reference, having its own tracker, may be used to register the femur coordinate system FBONE and tibia coordinate system TBONE to the bone tracker coordinate systems BTRK1 and BTRK2, respectively. Given the fixed relationship between the bones and their bone trackers 44, 46, positions and orientations of the femur F and tibia T in the femur coordinate system FBONE and tibia coordinate system TBONE can be transformed to the bone tracker coordinate systems BTRK1 and BTRK2 so the localizer 34 is able to track the femur F and tibia T by tracking the bone trackers 44, 46. These pose-describing data can be stored in memory integral with both manipulator controller 54 and navigation controller 26.

The working end of the surgical tool 22 has its own coordinate system. In some implementations, the surgical tool 22 comprises a handpiece and an accessory that is removably coupled to the handpiece. The accessory may be referred to as the energy applicator and may comprise a bur, an electrosurgical tip, an ultrasonic tip, or the like. Thus, the working end of the surgical tool 22 may comprise the energy applicator. The coordinate system of the surgical tool 22 is referenced herein as coordinate system EAPP. The origin of the coordinate system EAPP may represent a centroid of a surgical cutting bur, for example. In other implementations, the accessory may simply comprise a probe or other surgical tool with the origin of the coordinate system EAPP being a tip of the probe. The pose of coordinate system EAPP is registered to the pose of base tracker coordinate system BATR before the procedure begins (see transforms T1, T2, T3). Accordingly, the poses of these coordinate systems EAPP, BATR relative to each other are determined. The pose-describing data can be stored in memory integral with both manipulator controller 54 and navigation controller 26.

Referring to FIG. 2, a localization engine 100 is a software module that can be considered part of the navigation system 20. Components of the localization engine 100 run on navigation controller 26. In some implementations, the localization engine 100 may run on the manipulator controller 54. Localization engine 100 receives as inputs the signals from the localizer 34 and, in some implementations, signals from the tracker controller. Based on these signals, localization engine 100 can determine the pose of the bone tracker coordinate systems BTRK1 and BTRK2 in the localizer coordinate system LCLZ (see transform T6). Based on the same signals received for the base tracker 48, the localization engine 100 determines the pose of the base tracker coordinate system BATR in the localizer coordinate system LCLZ (see transform T1).

The localization engine 100 forwards the signals representative of the poses of trackers 44, 46, 48 to a coordinate transformer 102. Coordinate transformer 102 is a navigation system software module that runs on navigation controller 26. Coordinate transformer 102 references the data that defines the relationship between the preoperative images of the patient and the bone trackers 44, 46. Coordinate transformer 102 can also store the data indicating the pose of the working end of the surgical tool 22 relative to the base tracker 48.

During the procedure, the coordinate transformer 102 receives the data indicating the relative poses of the trackers 44, 46, 48 to the localizer 34. Based on these data, the previously loaded data, and the below-described encoder data from the manipulator 12, the coordinate transformer 102 can generate data indicating the relative positions and orientations of the coordinate system EAPP and the bone coordinate systems, FBONE and TBONE. As a result, coordinate transformer 102 generates data indicating the position and orientation of the working end of the surgical tool 22 relative to the tissue (e.g., bone) against which the working end is applied. Image signals representative of these data are forwarded to displays 28, 29 enabling the surgeon and staff to view this information. In certain implementations, other signals representative of these data can be forwarded to the manipulator controller 54 to guide the manipulator 12 and corresponding movement of the surgical tool 22.

The manipulator 12 has the ability to operate in a manual mode or a semi-autonomous mode in which the surgical tool 22 is moved along a predefined tool path, as described in U.S. Patent No. 9,119,655, entitled, "Surgical Manipulator Capable of Controlling a Surgical Instrument in Multiple Modes," the disclosure of which is hereby incorporated by reference, or the manipulator 12 may be configured to move in the manner described in U.S. Patent No. 8,010,180, entitled, "Haptic Guidance System and Method", the disclosure of which is hereby incorporated by reference.

The manipulator controller 54 can use the position and orientation data of the surgical tool 22 and the patient's anatomy to control the manipulator 12 as described in U.S. Patent No. 9,119,655, entitled, "Surgical Manipulator Capable of Controlling a Surgical Instrument in Multiple Modes," the disclosure of which is hereby incorporated by reference, or to control the manipulator 12 as described in U.S. Patent No. 8,010,180, entitled, "Haptic Guidance System and Method", the disclosure of which is hereby incorporated by reference.

The manipulator controller 54 may have a central processing unit (CPU) and/or other manipulator processors, memory, and storage. The manipulator controller 54, also referred to as a manipulator computer, is loaded with software as described below. The manipulator processors could include one or more processors to control operation of the manipulator 12. The processors can be any type of microprocessor or multi-processor system. The term processor is not intended to limit any implementation to a single processor.

A plurality of position sensors are associated with the plurality of links 58 of the manipulator 12. In one implementation, the position sensors are encoders. The position sensors may be any suitable type of encoder, such as rotary encoders. Each position sensor is associated with a joint actuator, such as a joint motor. Each position sensor is a sensor that monitors the angular position of one of six motor driven links 58 of the manipulator 12 with which the position sensor is associated. Multiple position sensors may be associated with each joint of the manipulator 12 in some implementations. The manipulator 12 can also include a force/torque sensor coupled between the distal end of the manipulator 12 and the end effector for detecting manual forces/torques exerted on the tool 22 by an operator. The input forces/torques can be used to command movement of the manipulator 12 and/or to detect collisions with the tool 22.

In some modes, the manipulator controller 54 determines the desired location to which the surgical tool 22 should be moved. Based on this determination, and information relating to the current location (e.g., pose) of the surgical tool 22, the manipulator controller 54 determines the extent to which each of the plurality of links 58 needs to be moved in order to reposition the surgical tool 22 from the current location to the desired location. The data regarding where the plurality of links 58 are to be positioned is forwarded to joint motor controllers JMCs that control the joints of the manipulator 12 to move the plurality of links 58 and thereby move the surgical tool 22 from the current location to the desired location. In other modes, the manipulator 12 is capable of being manipulated as described in U.S. Patent No. 8,010,180, entitled, "Haptic Guidance System and Method", the disclosure of which is hereby incorporated by reference, in which case the actuators are controlled by the manipulator controller 54 to provide gravity compensation to prevent the surgical tool 22 from lowering due to gravity and/or to activate in response to a user attempting to place the working end of the surgical tool 22 beyond a virtual boundary.

In order to determine the current location of the surgical tool 22, data from the position sensors is used to determine measured joint angles. The measured joint angles of the joints are forwarded to a forward kinematics module, as known in the art. Based on the measured joint angles and preloaded data, the forward kinematics module determines the pose of the surgical tool 22 in a manipulator coordinate system MNPL (see transform T3 in FIG. 3). The preloaded data are data that define the geometry of the plurality of links 58 and joints. With this encoder-based data, the manipulator controller 54 and/or navigation controller 26 can transform coordinates from the localizer coordinate system LCLZ into the manipulator coordinate system MNPL, vice versa, or can transform coordinates from one coordinate system into any other coordinate system described herein using transformation techniques. In many cases, the coordinates of interest associated with the surgical tool 22 (e.g., the tool center point or TCP), the virtual boundaries, and the tissue being treated, are transformed into a common coordinate system for purposes of relative tracking and display.

In the implementation shown in FIG. 3, transforms T1-T6 are utilized to transform relevant coordinates into the femur coordinate system FBONE so that the position and/or orientation of the surgical tool 22 can be tracked relative to the position and orientation of the femur (e.g., the femur model) and/or the position and orientation of the volume of material to be treated by the surgical tool 22 (e.g., a cut-volume model: see transform T10). The relative positions and/or orientations of these objects can also be represented on the displays 28, 29 to enhance the user's visualization before, during, and/or after surgery.

While the example surgical system 10 has been described with reference to the Figures, the surgical system 10 is not intended to be limited to what is specifically shown and described. For example, the surgical system 10 may not include the manipulator 12 or the navigation system 20 as specifically shown. Other systems are contemplated without departing from the scope of the disclosure.

### A. Head Mounted Device

Referring back to FIGS. 1 and 2, one or more head-mounted devices (HMDs) 200 may be included with the surgical system 10. The HMD 200 may be employed to enhance visualization before, during, and/or after surgery. The HMD 200 is an extended reality device, which can include aspects of augmented reality, mixed reality, virtual reality, and the like. The HMD 200 can be used to visualize the same objects previously described as being visualized on the displays 28, 29, and can also be used to visualize other objects, features, instructions, warnings, etc. The HMD 200 can be used to assist with visualization of surgical content, such as: medical imaging data, live stream surgical video, anatomical models, surgical procedure information, objects being tracked via the navigation system 20, instructions and/or warnings, among other uses, as described further below.

The HMD 200 has a display 208 onto which computer-generated content can be displayed onto a real-world view. In the implementation described herein, the HMD 200 provides on the HMD display 208 a computational holographic/superimposed/overlay of computer-generated content over the real-world view. In one example, the real-world view is acquired by a video camera 214 attached to the HMD. The video camera 214 produces a live video stream of the real-world and the computer-generated content may be combined into video stream of the real world. In such instances, the HMD display 208 may include one or more high-resolution displays positioned in front of the user's eyes. The HMD display 208 may be opaque in such scenarios.

In other implementations, the HMD 200 may implement natural see-through techniques whereby the HMD display 208 is implemented as a transparent lens/visor/waveguide provided between the user's eyes and the real-world. The real-world view is acquired naturally by the user's eyes, and the computer-generated content is provided on the transparent lens/visor/waveguide. Such see-through techniques can include a diffractive waveguide, holographic waveguide, polarized waveguide, reflective waveguide, or switchable waveguide.

The HMD 200 includes a support structure 202, which may be head-mountable in the form of an eyeglass or glasses, headwear or headset, or eyewear (such as a digital contact lens or lenses). The HMD 200 may include additional headbands or supports to hold the HMD 200 on the user's head. In other implementations, the HMD 200 may be integrated into a surgical helmet or other structure worn on the user's head, neck, and/or shoulders. Although not shown, it is contemplated that instead of the HMD 200, an extended reality display screen, such as a monitor, tablet, or hand-held display may be used, which can include similar hardware and capabilities as the HMD 200 described.

The HMD 200 can include an HMD controller 210. The HMD controller 210 can include a content generator 206 that generates the computer-generated content (also referred to as virtual images) and that transmits those images to the user through the HMD display 208. The HMD controller 210 controls the transmission of the computer-generated content to the HMD display 208. The HMD controller 210 may be a separate computer, located remotely from the support structure 202 of the HMD 200, or may be integrated into the support structure 202 of the HMD 200. The HMD controller 210 may be a laptop computer, desktop computer, microcontroller, or the like with memory, one or more processors (e.g., multi-core processors), input devices I, output devices (fixed display in addition to HMD 200), storage capability, etc.

The HMD 200 can include tracking sensors 212 that are in communication with the HMD controller 210. In some cases, the tracking sensors 212 are provided to establish a global coordinate system for the HMD 200, also referred to as an HMD coordinate system. The HMD coordinate system is established by these tracking sensors 212, which may comprise camera sensors or other sensor types, in some cases combined with IR depth sensors, to layout the space surrounding the HMD 200, such as using structure-from-motion techniques or the like. The HMD 200 can also comprise a photo/video camera 214 in communication with the HMD controller 210. The camera 214 may be used to obtain photographic images or video with the HMD 200, which can be useful in identifying objects or markers attached to objects, as will be described further below. The HMD 200 can comprise an inertial measurement unit IMU 216 in communication with the HMD controller 210. The IMU 216 may comprise one or more 3-D accelerometers, 3-D gyroscopes, and the like, to assist with determining a position and/or orientation of the HMD 200 in the HMD coordinate system or to assist with tracking relative to other coordinate systems. The HMD 200 could have a speaker to generate a sound or vibrate to provide an indication to the HMD user of a warning or other information of relevance.

The HMD 200 may also comprise control input sensors 217. In one example, the control input sensors 217 are configured to recognize biomechanical control input, such as gesture or eye-based commands from the user. When detecting hand-gestures, the control input sensor 217 is able to sense the user's hands, fingers, or other objects for purposes of determining the user's gesture command and controlling the HMD 200, HMD controller 210, navigation controller 26, and/or manipulator controller 54 accordingly. Gesture commands can be used for any type of input used by the system 10. The gesture commands may be detected below the HMD 200 or may be detected by the camera 214 in front of the HMD 200. The control input sensor 217 to detect gestures can include one or more cameras, infrared sensors, motion sensors, or the like. Gesture controls can include any type of hand or finger motion, including but not limited to: pinching, pointing, swiping, circling, grasping, twisting, or the like. When detecting eye-based commands, the control input sensor 217 is able to sense the user's eye position, motion, dwell time (stare), gaze and the like, for purposes of determining the user's intended command and controlling the HMD 200, HMD controller 210, navigation controller 26, and/or manipulator controller 54 accordingly. The eye-based commands may be detected using an eye-tracker that is positioned to face the user's eyes, e.g., in front of the HMD display 208. Eye-based controls can include any type of eye-command, including but not limited to: selecting an object, moving an object, or the like. In one example, the user can select a computer-generated object displayed by the HMD 200 by staring at the object continuously for a threshold amount of time. The HMD can also control input sensors 217 in the form of a microphone for recording verbal commands. The HMD controller 210 can process the verbal commands and control the HMD display 208 in response.

The HMD controller 210 can implement a decoder DEC. As will be described below, the decoder DEC can convert encoded video data or video streams that are remotely transmitted to the HMD 200 from another system. The decoder DEC can be any suitable type of decoder, such as a high efficiency video decoder (HEVC), multi-view high efficiency video decoder (MV-HEVC), VP9 decoder, AV1 decoder, and the like.

Any of the described components of the HMD 200 that can sense information or process sensed information (including but not limited to, the HMD controller 210, the video camera 214, tracking sensors 212, IMU 216, and/or control input sensors 217) can be understood as being part of a "sensing system" of the HMD 200. The sensing system is identified by numeral 219 in FIG. 2.

The HMD 200 can be registered to one or more objects used in the operating room, such as the tissue being treated, the surgical tool 22, the manipulator 12, the trackers 44, 46, 48, the localizer 34, and/or the like. In one implementation, a local coordinate system HMDCS is associated with the HMD 200 to move with the HMD 200 so that the HMD 200 is fixed in a known position and orientation in the HMD coordinate system. The HMD 200 can utilize the tracking sensors 212 to map the surroundings and establish the HMD coordinate system. The HMD 200 can then utilize the camera 214 to find objects in the HMD coordinate system. In some implementations, the HMD 200 uses the camera 214 to capture video images of markers attached to the objects and then determines the location of the markers in the local coordinate system HMDCS of the HMD 200 using motion tracking techniques and then converts (transforms) those coordinates to the HMD coordinate system.

In another implementation, a separate HMD tracker 218 (see FIGS. 2 and 3), similar to the trackers 44, 46, 48, could be mounted to the HMD 200 (e.g., fixed to the support structure 202). The HMD tracker 218 can have its own HMD tracker coordinate system HMDTRK that is in a known position/orientation relative to the local coordinate system HMDCS of the HMD 200. Alternatively, the tracker coordinate system HMDTRK could be calibrated to the local coordinate system HMDCS using calibration techniques. In this implementation, the local coordinate system HMDCS becomes the HMD coordinate system and the transforms T7 and T8 would instead originate therefrom. The localizer 34 could then be used to track movement of the HMD 200 via the HMD tracker 218 and transformations could then easily be calculated to transform coordinates in the local coordinate system HMDCS to the localizer coordinate system LCLZ, the femur coordinate system FBONE, the manipulator coordinate system MNPL, or other coordinate system.

Referring back to FIG. 3, a registration device 220 may be provided with a plurality of registration markers 224 (shown in FIG. 1) to facilitate registering the HMD 200 to the localizer coordinate system LCLZ. The HMD 200 locates the registration markers 224 on the registration device 220 in the HMD coordinate system via the camera 214 thereby allowing the HMD controller 210 to create a transform T7 from the registration coordinate system RCS to the HMD coordinate system. The HMD controller 210 then needs to determine where the localizer coordinate system LCLZ is with respect to the HMD coordinate system so that the HMD controller 210 can generate images having a relationship to objects in the localizer coordinate system LCLZ or other coordinate system. The registration device 220 or any technique for registering and/or calibrating the HMD 200 to another coordinate system can be like that described in US Patent No. 10,499,997, entitled "Systems and Methods for Surgical Navigation", the entire contents of which are hereby incorporated by reference in their entirety.

During use, for example, the localizer 34 and/or the navigation controller 26 can send data on an object (e.g., the cut volume model) to the HMD 200 so that the HMD 200 knows where the object is in the HMD coordinate system and can display an appropriate content in the HMD coordinate system. Any of the transforms T1-T12 can be combined to define or register the HMD coordinate system to any object. Once registration is complete, then the HMD 200 can be used to visualize computer-generated content in desired locations with respect to any objects in the operating room. Although these transforms have been described in detail, it is understood that the HMD 200 can operate without requiring any such transforms. The HMD 200 can display content without registering to the bone, or any part of the surgical system 10.

### B. Video Sources and Surgical Content

The surgical system 10 can include any number of surgical devices that include one or more video sources VS that are configured to generate video data VD including surgical content SC. In some cases, the video source VS can be a camera source. In other cases, the video source VS can be software or a computing device that presents video or from any source that can save or present video. The video can be pre-recorded or live stream video. The video data VD includes surgical content SC.

The surgical content SC can be live content (e.g., from the target site TS), or can be predetermined surgical content (e.g., surgical plan, anatomical measurements, anatomical models, etc.). The surgical content SC may include any information that may be relevant to the surgeon, patient, or surgical procedure. The surgical content SC may, but need not, be related to the process of actually performing surgery. The surgical content SC can be pre-operative surgical content SC. Alternatively, surgical content SC can include post-operative information, such as reports, etc. Examples of surgical content SC include but are not limited to: patient information, medical images (e.g., CT scan or volume, X-rays, etc.), surgical guidance information (e.g., tool interaction with target site), surgical planning information, an anatomical model, an implant model, a cut plan, a resection plan or volume, a virtual boundary VB or cutting boundary, surgical tool information, operating room or tool setup information, surgical step information, clinical application information, surgical alerts, notifications or warnings, and the like. The surgical content SC can be a step of the surgical procedure. The step of the surgical procedure can include but are not limited to: a pre-operative planning step, an operating room setup step, an anatomical registration step, an intra-operative planning step, an anatomical preparation step, or a post-operative evaluation step. The surgical content SC can include initialization, progression, or completion of any surgical step. Other examples of surgical content SC provided in the video data VD can include but are not limited to: location and/or detection of any surgical object (such as the bone, tracker, tool, robot or end effector, sensitive tissues, retractors, surgical table, imaging device, etc.), tool identification, anatomy information, surgical guidance information (e.g., tool interaction with target site), interaction between tools, amount of bone removed or needed to be removed, tool path TP, tool calibration, tool or component installation, surgical planning information, identification of an obstruction to a tool, line-of sight obstructions, surgeon ergonomics or posture, and the like. Further examples of video sources VS and surgical content SC are described below.

In one example, the surgical device can be localizer 34 and/or camera unit 36 of the navigation system 20. The video source VS1 can be the optical sensors 40 (visible light or machine vision camera) of the camera unit 36, which can generate video data VD of the surgical site. The surgical content can include a live webcam view of the surgical site or target site TS, for example. The navigation system 20 is also an example surgical device by virtue of the navigation controller 26 executing the clinical application CA. Here, the navigation controller 26 can be the video source VS2 by providing video data VD including stream that mirrors or duplicates representation of the clinical application CA. The surgical content SC in such examples can be anything presented by the clinical application CA. For example, the clinical application CA can have a plurality of different screens related to the surgical procedure. The screen can be a "Bone preparation," "pre-op check," "bone registration," "intra-op planning," "bone preparation," "case completion" or any other screen. The video data VD from the clinical application can include a guidance region that dynamically displays, in real-time, one or more of the surgical objects tracked by a localizer 34 of the navigation system 20. For example, the guidance region can display a graphical representation of the tracked surgical tool 22 relative to the target site TS to assist the surgeon in manipulating the target site TS.

Video data VD can be extracted from any other system/device (e.g., in the operating room) that is configured to display a software application. For example, the host system/device and software application can include any of: an endoscopic system that operates a software application for the endoscopic system; an imaging system (e.g., CT scanner) that operates a software application for the imaging system; a (CORE) console that operates a software application for operation of powered instruments; a surgical robot that operates a software application for controlling the surgical robot, a hand-held tool that operates a software application for controlling the hand-held tool, a surgical visualization system (e.g., arthroscope, ultrasound, laparoscope) that operates a software application for controlling the surgical visualization system, a surgical waste management system that operates a software application for controlling the surgical waste management system, a fluid management system that operates a software application for controlling the fluid management system, a sponge management system that operates a software application for controlling the sponge management system, a patient support apparatus that operates a software application for controlling the patient support apparatus, and the like.

Referring to FIG. 1, other video sources VS may include camera sources coupled to other surgical devices or surgical tools 22 used with the surgical system 10. For example, the surgical device can be a scope 27, such as but not limited to: an endoscope, a laparoscope, an arthroscope, and a microscope. The video source VS3 can be camera of the scope, and the video data VD can include live camera imagery/video of the target site TS produced by the scope 27. The scope 27 can be coupled to a control console 31 via a wired connection. The control console 31 can include a console controller 33 and communication system to communicate to the navigation system 20 using a wired or wireless connection. The control console 31 can also communicate with the HMD 200 using a wireless connection.

In other examples shown in FIG. 1, the surgical device can be the end effector 22 or manipulator 12 and the video source VS4 can be a camera coupled to the end effector 22 or manipulator 12. The camera can be coupled to, or adjacent to, the end effector 22, and implemented, for example, as described in U.S. Patent No. 10,531,926, entitled " Systems And Methods For Identifying And Tracking Physical Objects During A Robotic Surgical Procedure", the entire contents of which are hereby incorporated by reference. The camera can be coupled to other parts of the manipulator 12, such as at the base 57, or the like.

In another example, the surgical device can be a second HMD 200', e.g., worn by a second user. The second HMD 200' can include all the functionality and features of the HMD 200 described above. The video source VS can be a camera and/or the display of the second HMD 200', which can generate video data VD. The surgical content can include a first-person perspective view of the second HMD 200' user captured by the camera(s), a screen-sharing of the display of the second HMD 200', or the like.

Other videos sources may be in the operating room, such as a dedicated (standalone) camera (e.g., attached to a surgical boom or adjustable arm) utilized for viewing the operating room.

The disclosure is not limited to the example surgical devices and/or video sources that have been described. Other surgical devices and/or video sources are contemplated and may differ depending on the type of surgical procedure being performed or set up of the operating room. Moreover, the video data VD of any of the video sources VS can be processed by any suitable controller or computing system, depending on the system/device configuration. Such controllers/computing systems can include but are not limited to, the camera controller 42, the navigation controller 26, manipulator controller 54, tool controller, console controller 33, the HMD controller 210, or the like. Any of the video sources VS and any of the video data VD from the various video sources VS can be used individually or in combination. Any of the techniques described above can be used individually or in combination.

### C. Connectivity System

Referring to FIGS. 1 and 2, the system 10 may include a connectivity system or kit, CS, which communicates between the navigation system 20 or any of the described surgical devices with video sources VS and the HMD 200. The connectivity system CS is configured to receive any of the described video data VD from the video sources VS and perform modifications and/or evaluations to the video data VD in preparation for transmitting the video data VD to the HMD 200 for presentation.

In one example, the connectivity system CS includes a computing system (C), and an input device (ID) and output device (OD) and memory (M) coupled to the computing system C. The input device ID is configured to receive the video data VD form any of the described sources. The input device ID can be coupled to the video source VS using a wired input, such as a HMDI or DVI input. Conversion devices may be utilized to convert the format of the video data VD (e.g., converting from DVI to HMDI for example). As will be described below, the computing system C is configured to dynamically modify a quality of the video data VD in preparation for sending the video data VD to the HMD 200. The computing system C may implement a quality modifier QM to perform this function. The computing system C can implement an encoder ENC. As will be described below, the encoder ENC can encode the video data VD in preparation for remotely transmitting the video data VD to the HMD 200. The encoder ENC can be any suitable type of encoder, such as a high efficiency video encoder (HEVC), multi-view high efficiency video encoder (MV-HEVC), VP9 encoder, AV1 encoder, or the like.

The connectivity system CS can also include a communicator COM, which is configured to communicate with the HMD 200. The communicator COM can include any one or more devices that enable wireless communication. In one example, the communicator COM includes a wireless communication system, such as a WiFi router, Bluetooth transmitter, or the like. The HMD 200 is configured to communicate using the chosen communication method provided by the connectivity system CS. The output device OD may be the communicator COM itself, or the output device OD may be coupled to the communicator COM. The connectivity system CS may also be configured to receive any other type of data from the surgical device that provides the video source VS, such as control data, calibration data, or other information related to operation of the surgical device.

As shown in FIG. 1, the connectivity system CS can be a standalone device separate from any of the described surgical devices. The connectivity system CS can include a housing H that stores the various components of the connectivity system CS, including the computing system C and software, input device ID, memory M and communicator COM components. A mount MT can be attached to the housing H to enable the housing H to be mounted to any suitable location, such as a display or a component of a movable cart of the navigation system 20. For example, the mount MT can include a mounting bracket to fix to a host component or a mounting hook to hang the housing H onto a display.

In other implementations, the connectivity system CS can be integrated, in part, or in whole, into any of the surgical devices described, or navigation system 20. For example, the connectivity system CS can be implemented by the navigation controller 26 and the components of the connectivity system CS can be incorporated into the cart assembly 24. Also, the connectivity system CS can be integrated, in part, or in whole, into the HMD 200.

The connectivity system CS advantageously provides "plug and play" compatibility that surgeons and healthcare facilities demand. The connectivity system CS is well-adapted to be seamlessly compatible with existing surgical systems without significant re-development and re-design of the extended reality system and/or the surgical system. The connectivity system CS can be utilized to analyze or modify the video data VD of any video source VS provided by any manufacturer of surgical systems. The connectivity system CS can also communicate with any type of HMD that may be provided by any manufacturer of HMD systems The connectivity system CS provides information conversion capabilities between systems, even where such systems were not specifically developed to work together. In turn, the connectivity system CS can help ensure that the HMDs which are purchased by healthcare facilities or surgeons are compatible with the broad range of surgical systems and software required for various surgical procedures.

During or after the procedure, the connectivity system CS can transmit, to a remote server RS, any information from the system 10, such as the video data VD or information recognized from the video data VD or any contents that are displayed on the HMD 200. These contents can include video data VD transmitted to the HMD 200, video data VD produced by the HMD 200, any text or graphics detected within the video data VD and/or virtual objects that were displayed on the HMD 200. Other information can be logged, such as user inputs or behavior, system performance data, data transmission or performance, etc. The information can be transmitted for post-operative data analytic purposes or for improving future uses of the HMD 200. The remote server RS can be a cloud server or any suitable type of remote server. Multiple HMDs in the same facility or from multiple locations can communicate to the remote server RS. The remote server RS can include software for analyzing the information from the multiple HMDs to perform any of the described features. The remote server RS can also communicate software updates, calibration settings, or any other information described herein to any HMD 200.

### D. Displaying Video Data in HMD

As described above, the HMD 200 is configured to display video data VD that is generated by the video source VS, e.g., of a surgical device. Prior to being transmitted to the HMD 200, the video data VD can be processed by any suitable controller or computing system, depending on the system configuration. Such controllers/computing systems can include but are not limited to, the camera controller 42, the navigation controller 26, the connectivity system CS, the computing system C, manipulator controller 54, tool controller, or the HMD controller 210. Whatever the applicable system used to remotely process the video data VD is referred to in this section as the "controller(s)."

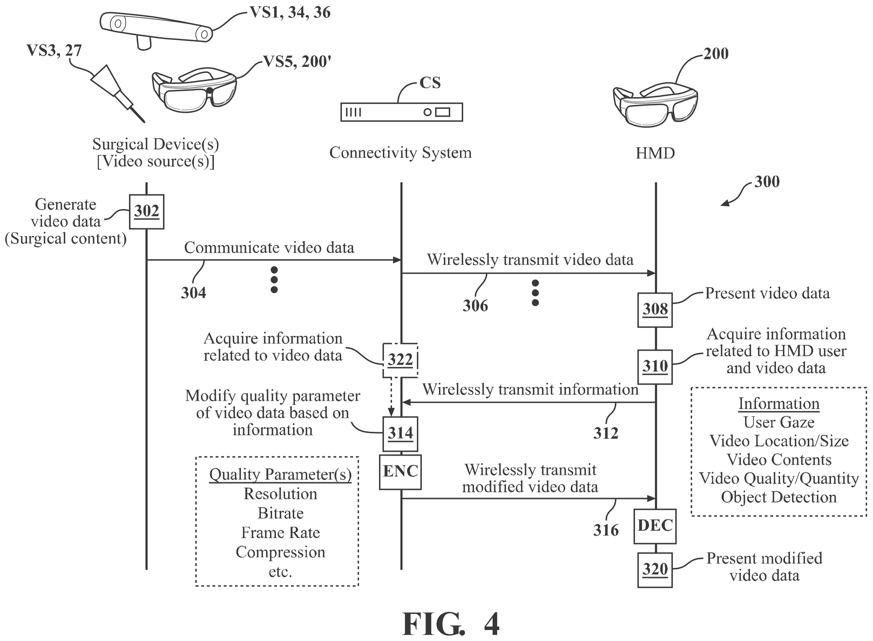

The HMD 200 is configured to receive the video data VD as a stream, whereby the video data is transmitted to the HMD 200 wirelessly over the internet in a continuous stream of data. The HMD controller 210 can utilize the decoder DEC to decode the video data VD prior to presentation. The HMD 200 can automatically display or be commanded to display, one or more virtual windows VW that include the video data VD. The virtual window VW can be combined with the real-world view. Multiple virtual windows VW can be presented on the HMD 200. The multiple virtual windows VW can be displayed in a side-by-side, nested, or overlapping manner. Examples of the virtual windows VW will be described in the subsequent section with reference to FIGS. 4-9.

The virtual window VW is presented relative to a field-of-view FOV of the HMD display 208. If the display 208 is an opaque display positioned in front of the user's eyes, the field-of-view FOV of the HMD display 208 is the extent of the displayed area in front of the user that the user can see with or on the HMD display 208. In one example, the FOV has a resolution of 4K. If the display 208 is transparent, the field-of-view FOV of the HMD display 208 is the extent of the real-world area that the HMD user can see through the HMD display 208. The virtual window VW may be a sub-region of a field-of-view FOV of the HMD display 208. Alternatively, the virtual window VW may occupy an entirety of the field-of-view FOV of the HMD display 208 (e.g., full screen).

The virtual window VW can be presented at a user-defined or predetermined pose. In one example, presentation of the virtual window VW and/or the predetermined pose can be based on recognition of surgical steps or surgical content provided in the video data VD. The virtual window can be anchored, i.e., locked relative to various coordinate systems, such as the real-world coordinate system or HMD coordinate system. When anchored to the real-world coordinate system, the virtual window VW will remain in place as though it were fixed in the real world. For example, the virtual window VW will become smaller if the HMD user walks away from the virtual window VW or become larger if the HMD user walks towards the virtual window VW. In other examples, the virtual window VW can be anchored to the HMD coordinate system such that the virtual window VW will follow any head movements of the HMD user as though it were locked a predetermined distance from the user's eyes.

### II. Techniques For Remotely Modifying Video Data Quality To Optimize Streaming In Surgical Extended Reality

Having introduced the surgical system 10, HMD 200, and video sources VS above, this section now describes various systems, methods, software, and techniques involving dynamically modifying and/or optimizing quality of streaming of video content in surgical extended reality. The techniques described herein provide for dynamically modifying the quality of video content presented by the HMD 200 based on various conditions or situations. The implementation described herein overcome limitations of conventional surgical extended reality systems by providing a technical solution to address latency and limited bandwidth in streaming video in the operating room. The techniques described herein optimize bandwidth and/or optimize video quality parameters to consume significantly less bandwidth. The technical solutions described herein enable real-time (near-real time) streaming while maintaining quality of the video data VD to an extent customized to the user's visual experience. For instance, the solutions presented herein can examine the HMD user's virtual environment as the user consumes the video data VD, and therefore, can detect situations in which the video quality can be eased back without noticeably impacting the visual experience of the user. The advantages of this solution are further realized when more than one stream is sent to the same HMD or is streamed to multiple HMDs in the operating room. The above advantages are some of the benefits provided by the techniques described here and are not intended to limit the scope of the claimed subject matter nor identify key features or essential features of the claimed subject matter.

Turning to FIG. 4, a sequence diagram is illustrated to summarize steps of a method 300 of using the surgical system 10 to dynamically modify and/or optimize streaming of video content in surgical extended reality. Although the diagram in FIG. 4 illustrates certain devices performing certain functions, it should be understood that some of the devices may be combined with others and that the order or sequence of the diagram is not intended to be limiting. For example, as described, the connectivity system CS may be integrated with the surgical device or with the HMD 200. In other instances, the connectivity system CS may not be present, but instead the video source can be provided by a remote source of the video data VD, which may or may not be a surgical device. For example, the remote source could be the remote server RS. Additionally, the steps of FIG. 4 are not limited to the order shown and some steps may be implemented concurrently with other steps.

At 302, the video source VS of any of the described surgical device(s) will generate video data VD. As described, this video data VD can include surgical content. At 304, the video data VD is communicated to the connectivity system CS. This communication can be through a direct wired connection (e.g., HDMI, DVI cable), for example, and hence may not be subjected to any latency or bandwidth issues.

At 306, the connectivity system CS wirelessly transmits the video data VD to the HMD 200. This step is described for illustrative purposes to explain that the transmission can include the video data VD before modification according to the techniques described herein, and therefore, the transmission may be subject to latency or bandwidth issues. However, it should be noted that this transmission may be at any time during the streaming process, including an initial transmission that includes modified video data VD according to the techniques described herein. At 308, the HMD 200 receives the transmitted video data VD and presents the virtual window VW that includes the video data VD presented therein. The virtual window VW can be presented in any manner described above.

### A. Acquisition of Information Related to User's Experience/Video Data

At 310, the HMD controller 210 can acquire or generate information related to the HMD user and the presented video data VD. For example, this information can be indicative of the user's experience in viewing, experiencing, or consuming the presented video data VD. In some cases, the information is actual data derived from the user's visual experience. In other cases, the information may be used to infer or predict what the user may be visually experiencing. In other cases, the information may not be related to the user, but not necessarily the user's visual experience. As will be described below, this information can be spatial information, contextual information, video qualitative and/or quantitative information, and the like. Any of the information sources can be utilized individually or in combination to implement the techniques described herein.

In one example, the HMD controller 210 can generate spatial information, which can relate to the relative size of the video data VD displayed to the user and/or to where the user is gazing at relative to the video data VD and/or HMD display 208. The spatial information can be related to the virtual window VW relative to the field-of-view FOV of the HMD display. For example, the HMD controller 210 can determine a size or area of the virtual window VW relative to the field-of-view FOV. The virtual window VW can occupy 10% or 50% of the entire field-of-view FOV, for example. The size of the virtual window VW can dynamically change as the HMD 200 moves. If so, the change in size can be dynamically computed by the HMD controller 210. In another example, the spatial information can be related to the gaze of the user relative to the virtual window VW. For example, the HMD controller 210 can identify that the gaze of the user is focused on the virtual window VW containing the video data VD and/or identify that the user is not focused on all other content presented on the HMD display 208. Conversely, the HMD controller 210 can identify that the gaze of the user is not focused on the virtual window VW containing the video data VD but instead is focused on content outside of the virtual window VW. If two virtual windows VW are presented which include different video data VD, the HMD controller 210 can identify which virtual window VW the user is focused on and which virtual window VW the user is not focused on. In another example, the HMD controller 210 can identify a sub-region of interest of the virtual window VW or video data VD that the user is focused on and/or identify a remaining region of the virtual window VW or video data VD that the user is not focused on.

In another example, the HMD controller 210 can generate contextual information based on the surgical contents of the video data VD. This contextual information can be acquired before or after presentation in the virtual window VW. To detect the contextual information, the HMD controller 210 is configured to detect in the video data VD any one or more of: a surgical step; an aspect of a surgical step; a portion or screen of a graphical user interface; a graphical element or icon, a surgical task requiring attention by the HMD user; a critical anatomical structure; presence of a surgical object or tool; an alert or warning, or the like. The HMD controller 210 can utilize image/graphic recognition algorithm(s) to identify any features of the video data VD to determine the context or video contents. For example, the HMD controller 210 can detect the difference between static imagery and live video. In another example, the HMD controller 210 can detect certain text/graphics that may be unique to the particular surgical procedure. For example, the HMD controller 210 may detect the word "tibia" to understand the context or contents of the video data VD, e.g., that this video data involves bone preparation for the tibia (as compared to the femur, for example). The HMD controller 210 can have certain words stored in memory as being associated with the particular surgeries or steps of a procedure. Additionally, or alternatively, the HMD controller 210 may detect presence of particular objects in the video data VD, such as presence of a bone, implant, registration spheres, a tool, or any object. The HMD controller 210 can have certain graphics stored in memory or could implement machine learning models, such as convolutional neural networks to detect the context of the video data VD.

In another example, the HMD controller 210 can generate qualitative information related to the quality of the video data VD that the user is viewing. The qualitative information can be based on any parameter relate to video data transmission or presentation, including but not limited to: resolution, compression (level, size, ratio), bitrate, target bitrate, constant bitrate, variable bitrate, frame rate, resolution, group of picture (GOP) key frame size, profile and level, B-frame, reference frames, entropy coding, chroma subsampling, intra refresh, deblocking filter, tuning, encoding speed or the like. For example, the HMD controller 210 may detect that the video data VD is a 4K, 75FPS stream. Qualitative information can be combined with spatial information, for example, to identify that a particularly small sized virtual window VW has an exceedingly high resolution and/or low compression quality. The HMD controller 210 can also acquire quantitative information related to the video data VD, such as the duration a video has been playing, a quantity of virtual windows VW that are open or streaming content, the duration the user has been gazing at the video data VD or away from the video data VD, the duration of the user's session using the HMD generally, or the like. Performance information related to the performance of the video data VD can also be acquired. Performance data may relate to latency, buffering, stalling, or any of the qualitative or quantitative information described above.

The HMD controller 210 can generate all of the information described or can filter or limit the amount of information to acquire. Acquisition of certain information may be prioritized over other information. Acquisition of certain information may depend on the presence of other information. For example, the HMD controller 210 can prioritize acquisition of the spatial information such that the other types of information are not acquired unless there is some spatial information that is acquired first. The HMD controller 210 can employ a machine learning model trained on user interaction with video data in the extended reality environment to make predictions about what information should be acquired or what information would be most relevant to generate. The machine learning model may be a neural network, a deep learning model, reinforcement learning models, or the like.

At 312, the HMD 200 wirelessly transmits any of the acquired information (spatial, contextual, qualitative, quantitative) to the connectivity system CS. The HMD 200 can transmit acquired information at various times or depending on certain conditions. For example, the HMD 200 can transmit the information for each given frame of the video data VD or at any predetermined frequency, e.g., once three seconds. In another example, the HMD 200 can transmit the information in response to detection/presence of the information. The HMD 200 can transmit all the acquired information for a given moment or can filter or limit the amount of information to transmit depending on the circumstances.

### B. Remote Modification of Video Data Quality

At 314, the connectivity system CS receives the information (spatial, contextual, qualitative, quantitative) wirelessly transmitted by the HMD 200. The connectivity system CS utilizes the acquired information to modify at least one quality parameter of the video data VD. The connectivity system modifies the video data VD using the quality modifier QM implemented by one or more controllers (C) or processors on the connectivity system CS. After modification of the quality parameter(s) of the video data VD, the connectivity system CS can encode the video data using the encoder ENC in preparation for remotely transmitting the video data VD to the HMD 200. At 316, the connectivity system CS wirelessly transmits the modified video data VD to the HMD 200. Upon receipt, the HMD controller 210 can implement the decoder DEC to convert encoded video data VD. At 320, the HMD 200 presents the modified video data VD.

As described above, the HMD 200 transmits acquired information at certain times or based on certain conditions, such as for each given frame of the video data VD, at any predetermined frequency, or in response to detection of the information. Similarly, the connectivity system CS is configured to modify the quality parameter(s) of the video data VD for each given frame, at any predetermined frequency, or in response to receiving the detected information. The loop of steps involving acquisition of information at the HMD 200 (at 310), the transmission of the information to the connectivity system CS (at 312), the modification of the quality parameter(s) at the connectivity system CS (at 314), and the wireless transmission of the modified video data VD to the HMD 200 (at 316) can occur at discrete times or can occur continuously over time. Whether discrete or continuous, the modification of the quality parameter(s) is therefore dynamically implemented based on the acquired information at the HMD 200 to reflect real-time or near-real time user interaction with the video data VD.

The quality parameter(s) of the video data VD that is modified at 314 can be any one or more of: resolution, compression, bitrate, target bitrate, constant bitrate, variable bitrate, frame rate, resolution, group of picture (GOP) key frame size, profile and level, B-frame, reference frames, entropy coding, chroma subsampling, intra refresh, deblocking filter, tuning, encoding speed or the like.

The connectivity system CS can dynamically modify the quality parameter(s) of the video data VD in various manners, as will be described in further detail in the subsequent section. The quality modifications can be specifically implemented such that there is no perceptible difference in the HMD user's visual experience in consuming the video data VD. However, in other situations, some quality modifications may be perceptible to the user, but customed to not significantly affect the user's visual experience due to the determined irrelevance of such video data.

In one example, the connectivity system CS can receive spatial information related to a size of the virtual window VW relative to the field-of-view FOV of the HMD display 208. In response, the connectivity system CS can dynamically modify the quality parameter(s) based on the size of the virtual window VW. For example, the connectivity system CS can dynamically modify the quality parameter(s) to increase quality of the video data VD in response to detecting an increase in the size of the virtual window VW or decrease quality of the video data VD in response to detecting a decrease in the size of the virtual window VW. In another example, the resolution of the video data VD can be modified to be correlated to, proportional to, or depending on the size of the virtual window VW.

In another example, the connectivity system CS can receive spatial information related to the gaze of the user to identify that the gaze of the user is focused on a virtual window VW containing the video data VD and to identify that the user is not focused on the other content provided on the HMD display 208. In response, the connectivity system CS can dynamically modify the quality parameter(s) to increase quality of the video data VD in the virtual window VW. Conversely, the connectivity system CS can receive spatial information related to the gaze of the user to identify that the gaze of the user is focused on the other content and to identify that the user is not focused on the virtual window VW containing the video data VD. In response, the connectivity system CS can dynamically modify the quality parameter(s) to decrease quality of the video data VD in the virtual window VW.

In yet another example, the connectivity system CS can receive spatial information related to the gaze of the user to identify a sub-region of interest of one virtual window VW that the user is focused on and identify a remaining region of the virtual window VW that the user is not focused on. In response, the connectivity system CS can dynamically modify the quality parameter(s) to increase quality of the video data VD in the region of interest of the virtual window VW and/or dynamically modify the quality parameter(s) to decrease quality of the video data in the remaining region of the virtual window VW.

In some instances, the quality parameter(s) modification can be based on regions or sub-regions customized or shaped to specific content in the video data VD. For example, the quality of the video data VD can be modified for a surgical object detected in the video data VD. An outline or border of the surgical object can be delineated. The region of quality modification can be the region within the outline/border of the surgical object. This technique can utilize spatial and contextual information. The connectivity system CS can employ any suitable algorithm for determining the shape of an object in the video data VD. Such algorithms can include edge detection algorithms, shape modeling, active appearance modeling, statistical shape modeling, or the like. A machine learning model, such as a convolutional neural network can also be employed for shape or object detection.

For any of these examples, a threshold of change can be implemented by the connectivity system CS. The threshold can evaluate the information to determine if the quality parameter(s) should be changed. If the information indicates a minor change in the video data or user visual experience (e.g., gaze) below the threshold, then the connectivity system CS may maintain the video quality. Otherwise, if the change is above the threshold, the connectivity system CS can implement the quality parameter(s) modification. This threshold may be implemented to improve user visual experience by avoiding over-modification of the video data due to the fact that the HMD user may rapidly change their gaze or interaction with the video data VD.

The connectivity system CS may be configured to determine the most relevant quality parameter(s) to modify, a magnitude that the quality parameter(s) should be modified, a duration for which the quality parameter(s) should be modified, and the like. In one example, the connectivity system CS has a look-up table stored in memory (M) that relates certain acquired information to certain quality parameter modifications. For example, if the acquired information is spatial information that defines a specific area of the video data VD, the look-up table can provide a predetermined resolution associated with the specific area. In another examples, the connectivity system CS can employ a machine learning model trained on user interaction with video data in the extended reality environment to make predictions about what is the most relevant quality parameter to modify, a magnitude of the quality parameter should be modified, a duration for which the quality parameter should be modified, and the like. The machine learning model may be a neural network, a deep learning model, reinforcement learning models, or the like. The quality modifier QM can be implemented using a cost function optimization algorithm that modifies the quality parameter(s) in a manner that seeks to minimize data transmission (to the HMD) related to the video data VD while maintaining the user's visual experience in viewing the video data VD. For example, the optimization algorithm may be used to determine, based on spatial information related to the video data and HMD user, that the resolution of the video data can be reduced from 4K to 3K without impacting how the video data would be visually experienced by the HMD user. In some cases, the quality modifier QM can be equipped with prior data or a look-up table that includes viewing distances (of the HMD user relative to the video data size/location), HMD field-of-view size, and specified video quality parameters (e.g., resolution, etc.) to determine the most appropriate way to dynamically modify the video data quality for the detected condition. Conversely, if the viewing distance becomes closer and/or the video data size becomes larger, the quality modifier QM can increase the quality of the video data if the optimization algorithm determines that the benefits of a quality increase will become noticeable to the HMD user.

The duration of the quality parameter(s) modification can depend on the duration that the relevant information was acquired by the HMD 200. For example, if the HMD 200 acquired spatial information related to the user gazing at the video data VD for 5 seconds, the connectivity system CS can modify the quality parameter(s) of the video data VD for those 5 seconds and cease modification thereafter. Duration of modification can also depend on other factors such as the type of information, combination of information or higher order inferences about the user's experience, e.g., predicted by the machine learning model.

The magnitude of the quality parameter(s) modification can depend on factors such as the type of information, combination of information or higher order inferences about the user's experience, e.g., predicted by the machine learning model. For example, if the HMD 200 acquired spatial information related to the size of the virtual window VW being far away from the user's view on the HMD 200, the connectivity system CS can modify a magnitude of compression of the video data VD proportional to the virtual window VW size. The nature of the term "magnitude" in this context will depend on the specific quality parameter being modified. Magnitude can be substituted for scale, rate, size, speed, or the like.

Notably, another implementation is contemplated (as shown at 322 in FIG. 4) wherein the connectivity system CS itself can generate or extract information related to the video data VD. Such information can be contextual, qualitative, or quantitative information related to the video data VD. Due to the communication of the video data VD directly to the connectivity system CS, this capability can be performed in addition to, or instead of, the HMD 200 generating the information described above. Hence, the connectivity system CS can perform this function without necessarily requiring feedback from the HMD 200. In other words, the connectivity system CS need not wirelessly receive information from the HMD 200 at step 312. In turn, the connectivity system CS can proactively modify the quality parameter(s) of the video data VD, using a pass-through technique, in preparation for transmitting the modified video data VD to the HMD 200. This alternative implementation can be performed at discrete times, as a continuous pass-through, or can be performed before the HMD 200 ever receives the video data VD. The connectivity system CS can detect or generate any of this information in a manner similarly described in the above section in relation to the HMD 200. In one example, upon receiving the video data VD from the video source VS, the connectivity system CS can detect that the contents of the video data VD include a high priority surgical step that requires high resolution. In response, the connectivity system CS can utilize the quality modifier QM to dynamically increase the resolution of the video data VD so that the HMD user can visualize high resolution video of the surgical step. Upon detecting from the video data VD that the surgical step is no longer present, the connectivity system CS can cease increasing of the resolution. Additionally, or alternatively, the connectivity system CS may detect presence of particular objects in the video data VD, such as presence of a bone, implant, GUI elements, a tool, or any object. As described, the connectivity system CS can have certain graphics/shapes stored in memory or could implement machine learning models, such as convolutional neural networks or reinforcement learning to detect the context of the video data VD.

### C. Practical Examples

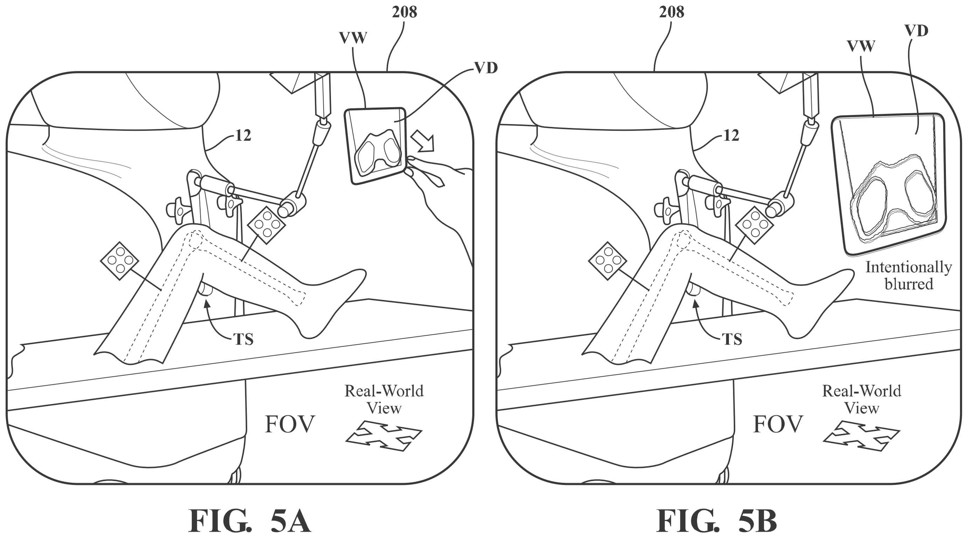

Described herein, and with reference to FIGS 5-9, are various practical examples of the techniques described above related to modification of the quality parameter(s) of the video data VD based on information detected from the video data VD and/or user experience in consuming the video data VD on the HMD display 208.

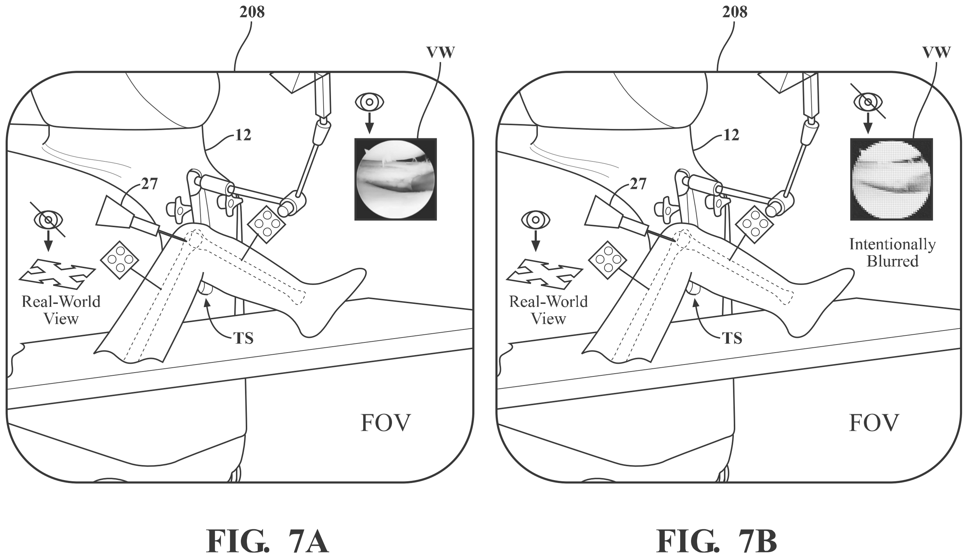

In each of the described and illustrated examples, a view of the HMD display 208 is illustrated from a first-person perspective of the HMD user, i.e., what the HMD user would see. In FIGS. 5-8, a real-world view of the operating room environment is shown. Again, the real-world view may be natural (see-through) view or reproduced video view, depending on the configuration of the HMD display 208. Where appropriate, the real-world view is indicated using a double arrow icon. In the examples of FIGS. 7-9, the user's gaze is indicated by an icon of an eye and the region that the user is not gazing is indicated by an icon of an eye being stricken through. These icons are for illustrative purposes and are not intended to be presented to the HMD user 208.

Also, certain images of the video data VD are intentionally blurred to visually symbolize quality modification(s) to the video data VD. The blurring has been provided for illustrative purposes and does not necessarily signify that the video data VD has been blurred. To reiterate, one advantage of the described techniques that any quality modification(s) to the video data VD would not be readily perceptible by the HMD user so as to maintain their visual experience.

### 1. Virtual Window Size

Referring to FIGS. 5A and 5B, one example is illustrated whereby the quality parameter(s) of the video data VD is modified based on a size of the virtual window VW relative to the field-of-view FOV of the HMD display 208. In this example, the HMD user is perceiving a real-world view of a portion of the surgical system 10 (including the manipulator 12 and patient/target site TS). The virtual window VW being streamed to the user on the HMD display 208 includes a reproduced/mirrored video from the clinical application CA of the navigation system 20. Of course, the type of video data VD could instead be real-time camera video data provided from any other video source VS or surgical device. In FIG. 5A, the virtual window VW is relatively far away from the user's view and hence exhibits a smaller size relative to the overall FOV size of the HMD display 208. Based on this spatial information, the quality parameter of the video data VD is maintained or increased. In this example, a resolution of the video data VD is maintained or increased based on its relative size. For example, the FOV may have a 4K resolution and the quality of the video data VD can be presented at 4K resolution because the benefits of 4K resolution would be perceptible to the HMD user at this distance/size.

In FIG. 5A, the user utilizes a gesture input (indicating by the illustrated hand) to grasp the virtual window VW and move the window VW closer to user's view. The result is the virtual window VW being enlarged in size in FIG. 5B. In FIG. 5B, the virtual window VW is relatively closer to the user's view and hence exhibits a larger size relative to the overall FOV size of the HMD display 208. Based on this spatial information, the quality parameter of the video data VD can be decreased. For example, the virtual window VW can be modified to display video data VD at a resolution of 3K resolution to maintain the user's experience in consuming the larger sized video data VD. The resolution modification can result in no perceptible difference in the user's experience due to the size of the window VW.

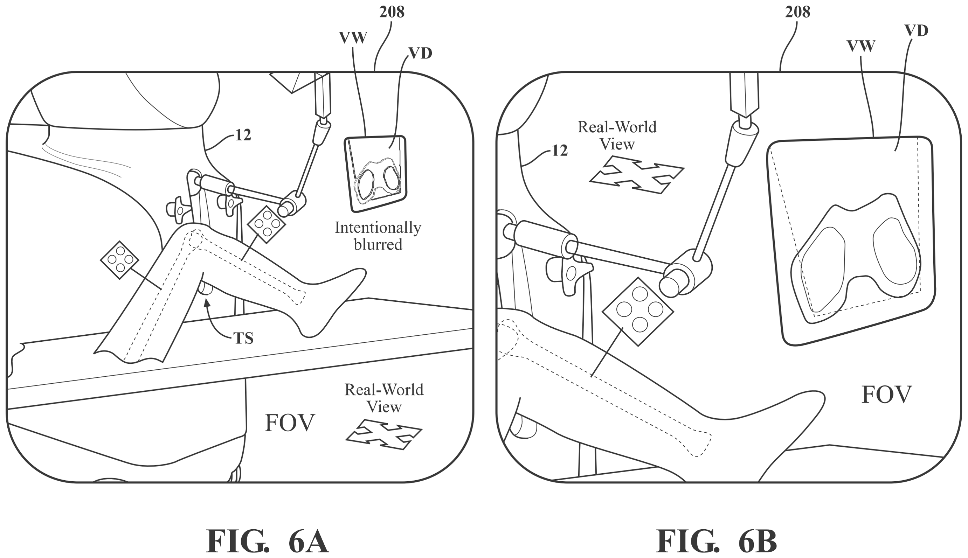

FIGS. 6A and 6B illustrate a similar example, but here the virtual window VW is anchored in the real-world coordinate system, e.g., above the patient's leg. In FIG. 6A, the HMD user is standing relatively far away from the virtual window VW, which results in a correspondingly smaller sized virtual window VW relative to the overall FOV size of the HMD display 208. Based on this spatial information, the quality parameter of the video data VD is reduced. In FIG. 6A, a compression of the video data VD is increased, and a bitrate of the video data VD is reduced based on the geometry of the virtual window VW relative to the FOV. In FIG. 6B, the HMD user moves closer to the anchor virtual window VW resulting in movement of the window VW closer to user's view and enlargement of the window size. The spatial information related to size of the window VW relative to the overall FOV size of the HMD display 208 is utilized to modify the quality parameter to display video data VD at a lower compression rate and at a higher bit rate to maintain the user's experience in consuming the larger sized video data VD.

### 2. User's Gaze Focused At/Away From Video Data

Referring to FIGS. 7A and 7B, another example is illustrated whereby the quality parameter(s) of the video data VD is modified based on where the HMD user is looking at relative to the overall FOV of the HMD display 208. In this example, the HMD user is once again perceiving a real-world view of a portion of the surgical system 10. The real-world view is provided by a video representation of the real-world captured by the camera(s) 214 of the HMD 200. The virtual window VW being streamed to the user on the HMD display 208 includes real-time video data VD generated by the camera of the scope 27 provided at the surgical site. In FIGS. 7A and 7B, the virtual window VW exhibits the same size relative to the overall FOV size of the HMD display 208. However, in FIG. 7A, the HMD controller 210 detects that the user's gaze is directed at the virtual window VW and not the environment surrounding the virtual window VW. Spatial information is acquired about the user's gaze and the coordinates of the gaze relative to the FOV and/or window VW. Based on this spatial information, the quality parameter of the video data VD may be maintained or increased and the quality of the surrounding environment (which is not being gazed upon) can remain unmodified. In some cases where the video of the surrounding environment is not captured by the cameras of HMD 200, but from the video source, the quality of the surrounding environment (which is not being gazed upon) can be temporarily decreased. In FIG. 7A, let us suppose the video data VD is presented with a 4K resolution at a 60 Mbps bitrate.

In FIG. 7B, the HMD controller 210 detects that the user's gaze has changed and is now directed at the real-world environment surrounding the virtual window VW. Based on spatial information related to the user's gaze, the quality parameter of the video data VD (which is not being gazed upon) may be decreased. For example, the video data VD quality may be decreased to 3K resolution at a 40Mbps bitrate. Meanwhile, the quality of the surrounding environment can be maintained or remain unaltered. Again, however, if the scenario is that the video of the surrounding environment is not captured by the cameras of HMD 200, but from the video source, the quality of the surrounding environment (which is being gazed upon) can be temporarily increased. Because the live-camera video stream is bandwidth intensive, reducing the quality of the video data VD can provide the benefits of reducing latency and improving streaming performance of the HMD 200 without any noticeable difference to the user's visual experience.

### 3. User's Gaze Relative to Multiple Virtual Windows

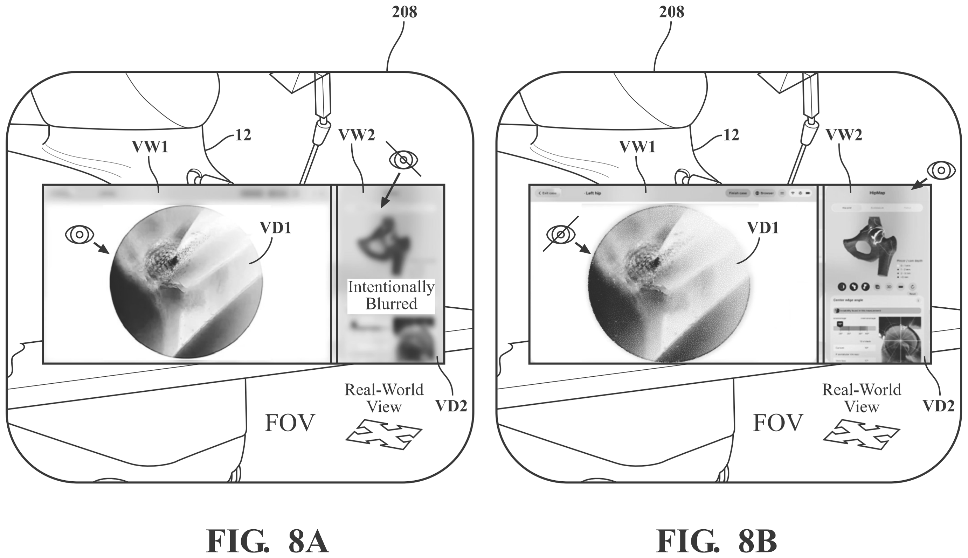

Referring to FIGS. 8A and 8B, another example is illustrated whereby the quality parameter(s) of the video data VD is modified based on where the HMD user is looking at relative to two virtual windows VW1, VW2 presented on the HMD display 208. Each virtual window VW1, VW2 presents distinct video data VD1, VD2. For example, the first virtual window VW1 is presenting real-time video data VD generated by the camera of the scope 27 provided at the surgical site. The second virtual window VW2 is located adjacent to the first window and presents a mirrored stream of a portion of a graphical user interface GUI from a clinical application CA. In this example, the HMD user is once again perceiving a real-world of the operating room provided by a video representation of the real-world captured by the camera(s) 214 of the HMD 200.

In FIG. 8A, the HMD controller 210 detects that the user's gaze is directed at the first virtual window VW1 and not second virtual window VW2. In turn, the HMD controller can generate information 210 to identify that the first virtual window VW1 is the active window and that the second virtual window VW2 is an inactive window. Spatial information can be acquired about the user's gaze and the coordinates of the gaze relative to the FOV and/or first window VW1. Additionally, contextual information can be generated to identify that the first virtual window VW1 is a live-camera view and hence has a higher priority than the second window VW2. Based on this acquired information, the quality parameter of the first video data VD1 in the first virtual window VW1 may be maintained or increased and the quality of the second video data VD2 of the second window VW2 (which is not being gazed upon) can be temporarily decreased. In this example of FIG. 8A, the resolution and compression of the first video data VD1 is maintained. However, for the second video data VD2, the resolution is reduced, and the compression is increased.

In FIG. 8B, the HMD controller 210 detects that the user's gaze has changed and is now directed at the second virtual window VW2 causing the first virtual window VW1 to be the inactive window and the second virtual window VW2 to be the active window. Once again, spatial information and/or contextual information can be generated resulting in the resolution and compression of the second video data VD2 being maintained. For the second video data VD2, the resolution is reduced, and the compression is increased resulting in a lower quality. However, based on the priority of the second video data VD2 identified by virtue of the contextual data, the quality reduction of the second video data VD2 is intentionally selected so as to not fully disrupt the live-camera stream.

### 4. User's Gaze Relative to Sub-Region of Video Data

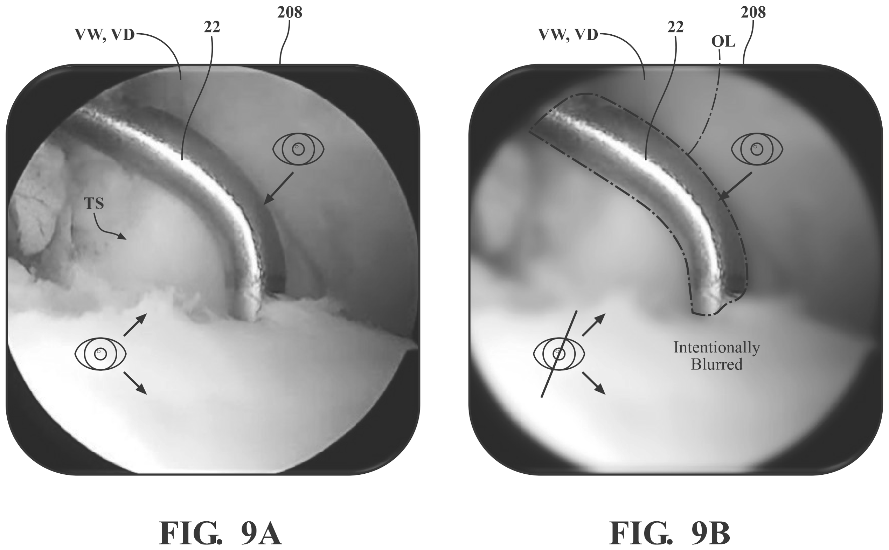

Referring to FIGS. 9A and 9B, yet another example is illustrated whereby the quality parameter(s) of the video data VD is modified based on a sub-region that the HMD user is looking at relative to the video data VD. In this example, one virtual window VW is provided in a full screen format and presents real-time video data VD generated by the camera of the scope 27. The video data VD contains an image of a surgical tool 22 interacting with the target site TS.

In FIG. 9A, the HMD controller 210 detects that the user's gaze is moving rapidly between the full view of the video data VD. The user is focused on both the target site TS and the tool 22. Contextual information can be generated to identify that the video data VD contains a representation of the surgical tool 22. Spatial information can be acquired about the user's gaze. However, the quality modifier QM can determine that the gaze does not meet the threshold for modifying quality of any aspect of the video data VD. Based on this determination, the quality parameter of the video data VD in the virtual window VW may be maintained

In FIG. 9B, the HMD controller 210 detects that the user's gaze has changed and is now heavily focused on the surgical tool 22 and not the surrounding target site TS. Based on the spatial information related to the gaze and contextual information related to the surgical tool 22, the surgical tool 22 can be identified as an active sub-region or active object. The outline OL of the surgical tool 22 is identified using the described algorithm(s). As a result, the resolution of the tool 22 within the outline OL region is maintained. Meanwhile, for the region outside of the tool 22 outline OL, the resolution is reduced for a temporary period until the user's gaze shifts to the overall view.

In other examples, instead of the sub-region being shaped to a detected object, the sub-region may be a foveated region of focus based on the gaze at the expense of regions beyond the line-of-sight of the user. It should be appreciated however that the techniques described herein differ from prior techniques related to foveated rendering due to the quality modification occurring (in some implementations) remotely (e.g., with the connectivity system), rather than being implemented solely by software/hardware of the HMD 200, thereby reducing latency involved with streaming the video data before it reaches the HMD 200.

By implementing the quality modification of the video data VD remote from the HMD 200, the techniques described herein robustly address latency and limited bandwidth in wirelessly streaming video on the HMD 200 in the operating room. Since lower quality video data VD is transmitted from the remote source, the techniques described herein optimize bandwidth to consume significantly less bandwidth while maintaining quality of the video data VD to an extent customized to the user's experience.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

## Claims

1. A surgical system (10) comprising:
a surgical device (22, 27, 34, 36, 200') that includes a video source (VS) that is configured to generate video data (VD) including surgical content (SC);
a head-mounted device (HMD)(200) comprising an HMD controller (210), and an HMD display (208) positionable in front of a user's eyes; and
a connectivity system (CS) in communication with the video source (VS) and being remote from the HMD (200), the connectivity system (CS) being configured to wirelessly communicate with the HMD controller (210) and wirelessly transmit the video data (VD) to the HMD controller (210);
wherein the HMD controller (210) is configured to:
present, on the HMD display (208), a virtual window (VW) containing the video data (VD);
acquire spatial information related to one or both of: the virtual window (VW) relative to a field-of-view (FOV) of the HMD display (208); and a gaze of the user relative to the virtual window (VW); and
wirelessly transmit the spatial information to the connectivity system (CS); and
wherein the connectivity system (CS) is configured to:
receive the spatial information acquired by the HMD controller (210);
dynamically modify at least one quality parameter of the video data (VD) based on the spatial information to generate modified video data (VD); and
wirelessly transmit the modified video data (VD) to the HMD controller (210) for presentation on the HMD display (208).

2. The surgical system (10) of claim 1, wherein the at least one quality parameter of the video data (VD) comprises one or more of: a resolution, a bitrate, a frame rate; and a compression parameter of the video data (VD).

3. The surgical system (10) of any preceding claim, wherein the connectivity system (CS) further dynamically modifies the at least one quality parameter based on an optimization algorithm that seeks to minimize data transmission related to the video data (VD) while maintaining the user's experience in viewing the video data (VD).

4. The surgical system (10) of any preceding claim, wherein:
the spatial information is related to a size of the virtual window (VW) relative to the field-of-view (FOV) of the HMD display (208); and
the connectivity system (CS) is configured to dynamically modify the at least one quality parameter of the video data (VD) based on the size of the virtual window (VW) relative to the field-of-view (FOV).

5. The surgical system (10) of claim 4, wherein:
the at least one quality parameter comprises a resolution of the video data (VD); and
the connectivity system (CS) is configured to dynamically modify the resolution of the video data (VD) proportional to the size of the virtual window (VW).

6. The surgical system (10) of any preceding claim, wherein the spatial information is related to the gaze of the user relative to the virtual window (VW).

7. The surgical system (10) of claim 6, wherein:
the spatial information indicates a sub-region of interest of the virtual window (VW) that the user is focused on and indicates a remaining region of the virtual window (VW) that the user is not focused on; and
based on the spatial information, the connectivity system (CS) is configured to:
dynamically modify the at least one quality parameter to increase quality of the video data (VD) in the sub-region of interest of the virtual window (VW); and
dynamically modify the at least one quality parameter to decrease quality of the video data (VD) in the remaining region of the virtual window (VW).

8. The surgical system (10) of claim 6, wherein:
the spatial information indicates an object of interest of the virtual window (VW) that the user is focused on and indicates a remaining region of the virtual window (VW) that the user is not focused on; and
based on the spatial information, the connectivity system (CS) is configured to dynamically modify the at least one quality parameter of the video data (VD) in a region defining the object of interest of the virtual window (VW).

9. The surgical system (10) of claim 6, wherein:
the HMD controller (210) is configured to present, on the HMD display (208), other content outside of the virtual window (VW) containing the video data (VD);
the spatial information indicates that the gaze of the user is focused on the virtual window (VW) containing the video data (VD) and indicates that the user is not focused on the other content; and
based on the spatial information, the connectivity system (CS) is configured to dynamically modify the at least one quality parameter to increase quality of the video data (VD) in the virtual window (VW);
or
the spatial information indicates that the gaze of the user is focused on the other content and indicates that the user is not focused on the virtual window (VW) containing the video data (VD); and based on the spatial information, the connectivity system (CS) is configured to dynamically modify the at least one quality parameter to decrease quality of the video data (VD) in the virtual window (VW).

10. The surgical system (10) of any preceding claim, wherein:
the HMD controller (210) is configured to present, on the HMD display (208), the virtual window (VW) containing the video data (VD) according to a frame rate of at least 60 Hz; and
the connectivity system (CS) is configured to receive the spatial information and dynamically modify the at least one quality parameter of the video data (VD) based on the spatial information, for each frame.

11. The surgical system (10) of any preceding claim, wherein the surgical device (22, 27, 34, 36, 200') comprises one of: a surgical scope (27) comprising a camera as the video source (VS); a surgical robot (12) or surgical tool (22) comprising a camera as the video source (VS); a navigation system (20) comprising a camera (36) as the video source (VS); a second HMD (200') comprising a camera as the video source (VS); an ultrasound scanner coupled to the video source (VS); or a navigation system (20) that executes a clinical application (CA) that provides the video data (VD).

12. The surgical system (10) of any preceding claim, wherein the connectivity system (CS) is configured to:
detect information about the video data (VD), the information comprising contextual information, quantitative information and/or qualitative information about the video data (VD); and
dynamically modify the at least one quality parameter of the video data (VD) based further on the detected information.

13. A method (300) of operating the surgical system (10) of any preceding claim.

14. A connectivity system (CS) for use with a surgical system (10), the surgical system (10) comprises a surgical device (22, 27, 34, 36, 200') that includes a video source (VS) to generate video data (VD) including surgical content (SC), a head-mounted device (HMD)(200) with an HMD controller (210) and an HMD display (208) positionable in front of a user's eyes, the connectivity system (CS) comprising:
a housing (H) located remote from the HMD (200);
a controller (C) disposed within the housing (H) and being configured to:
receive the video data (VD) from the video source (VS);
wirelessly transmit the video data (VD) to the HMD controller (210) for presentation, on the HMD display (208), of a virtual window (VW) containing the video data (VD);
wirelessly receive spatial information from the HMD controller (210), the spatial information related to one or both of: the virtual window (VW) relative to a field-of-view (FOV) of the HMD display (208); and a gaze of the user relative to the virtual window (VW);
dynamically modify at least one quality parameter of the video data (VD) based on the spatial information to generate modified video data (VD); and
wirelessly transmit the modified video data (VD) to the HMD controller (210) for presentation on the HMD display (208).

15. A head-mounted device (HMD)(200) for use with a surgical system (10), the surgical system (10) comprises a surgical device (22, 27, 34, 36, 200') that includes a video source (VS) to generate video data (VD) including surgical content (SC), and a connectivity system (CS) in communication with the video source (VS), the HMD (200) comprising:
a structure (202) to be worn on a head of a user;
an HMD display (208) supported by the structure (202) and positionable in front of a user's eyes; and
an HMD controller (210) being configured to:
wirelessly communicate with the connectivity system (CS) to receive the video data (VD);
present, on the HMD display (208), a virtual window (VW) containing the video data (VD);
acquire spatial information related to one or both of: the virtual window (VW) relative to a field-of-view (FOV) of the HMD display (208); and a gaze of the user relative to the virtual window (VW);
wirelessly transmit the spatial information to the connectivity system (CS);
wirelessly receive modified video data (VD) from the connectivity system (CS), the modified video data (VD) having been altered by the connectivity system (CS) through dynamic modification of at least one quality parameter of the video data (VD) based on the spatial information; and
present the modified video data (VD) on the HMD display (208).
